# EUROPEAN PATENT SPECIFICATION

(11) **EP 0 708 667 B1**
(45) Date of publication and mention of the grant of the patent: **24.10.2001**
(21) Application number: 93917169.0
(22) Date of filing: 14.07.1993
(51) Int. Cl.: A61M 5/32

(54) **SAFETY HYPODERMIC NEEDLE**
HYPODERMISCHE SICHERHEITSNADEL
AIGUILE HYPODERMIQUE DE SECURITE

(43) Date of publication of application: 01.05.1996
(73) Proprietor: Poulsen, Thomas Edward, La Moille, NV 89828 (US)
(72) Inventor: Poulsen, Thomas Edward, La Moille, NV 89828 (US)
(74) Representative: Quest, Barry
(86) International application number: PCT/US93/06612
(87) International publication number: WO 95/02427

(56) References cited:
- EP-A- 0 299 287
- EP-A- 0 409 180
- EP-A- 0 513 869
- WO-A-91/07196
- DE-U- 9 007 104
- GB-A- 2 248 021
- US-A- 4 795 432
- US-A- 4 804 371
- US-A- 4 850 977
- US-A- 4 863 435
- US-A- 4 890 996
- US-A- 4 921 490
- US-A- 4 927 416
- US-A- 5 176 856

## Description

### BACKGROUND OF THE INVENTION

One of the major safety concerns of nurses and other health workers is the risk of accidentally pricking oneself with contaminated hypodermic needles. This concern has become particularly acute since the onset of the AIDS epidemic. It has been reported that, as of January 1989, 20 health workers have been infected by the AIDS virus by such accidental needle pricks.

In light of the dangers associated with contaminated hypodermic needles, it would be of importance to develop a hypodermic needle that is safer than existing hypodermic needles to the people who must handle them. Accordingly, it is an object of the present invention to provide a hypodermic needle that is safer than existing hypodermic needles for health workers and others to use and handle. In keeping with the proceeding object, it is also an object of this invention to develop such a hypodermic needle that is not cumbersome in use, and that can be put into the safe mode by a simple action by the health worker immediately after the needle has been removed from the patient. And in light of the large quantity of hypodermic needles that are used by the health profession, and the expense involved with this large quantity of use, it is a further object of this invention to provide such a safe, easy to use hypodermic needle that can be manufactured relatively inexpensively.

### SUMMARY OF THE INVENTION

In the several embodiments, the invention includes a hypodermic needle with a hollow member positioned over the needle segment of the hypodermic needle. The hollow member covers a portion of the needle, and is capable of sliding along the needle. Prior to and while the hypodermic needle is being used, the hollow member is held away from the piercing end of the needle, thus enabling the needle to be used in the normal manner. After the needle has become contaminated, the hollow member is slid forward along the needle to cover its piercing end. The hollow member is supported in a position covering the piercing end of the needle against rearward pressure, and is similarly prevented from sliding off the end of the needle. In several of the embodiments, the hollow member is pushed forward by a spring, thus requiring only a simple action by the user to put the hypodermic needle in a safe condition for subsequent handling.

### BRIEF DESCRIPTION OF THE DRAWINGS

Fig. 1 is a side view of an ordinary hypodermic needle.

Fig. 2 is a perspective view of the hypodermic needle of fig. 1, showing the opening for receiving the syringe or I.V. attachment.

Fig. 3 is a side view of the first embodiment of the invention as it would appear before it has been used.

Fig. 4 is a side view of the embodiment of fig. 3, showing the hypodermic needle after it has been put into the safe mode after use.

Fig. 5 shows a variation of the embodiment shown in figs. 3 and 4.

Fig. 6 is a side view of the second embodiment of the invention shown before use.

Fig. 7 is a side view of the third embodiment of the invention before use.

Fig. 8 is a side view of the fourth embodiment of the invention before use.

Fig. 9 is a partial side view of the embodiment of fig. 8, showing a variation of that embodiment.

Fig. 10 is a partial view, from the top, of the embodiment of fig. 8, showing a variation of that embodiment.

Fig. 11 is a side view of the fifth embodiment of the invention shown before use.

Fig. 12 shows the embodiment of fig. 11 with a longer needle, and, in one alternate, a longer hub.

Fig. 13 is a partial view, from the top, of the embodiment of figs. 11 and 12, showing a variation of that embodiment.

Fig. 14 is a partial side view of the embodiment of figs. 11 and 12, showing a variation of that embodiment.

Fig. 15 is a side view of the sixth embodiment of the invention before use.

Fig. 16 is a side view of the seventh embodiment of the invention before use.

Fig. 17 is a side view of the eighth embodiment of the invention before use.

Fig. 18 shows a variation of the embodiment shown in fig. 17.

Fig. 19 shows the embodiment of fig. 17 with a longer needle.

Fig. 20 shows the ninth embodiment of the invention in which a hypodermic needle and a syringe, which is shown in part, are combined as a single functioning unit.

Fig. 21, the tenth embodiment, shows the embodiment of fig. 11 with which a specially designed syringe, shown in part, is used for ease of operation.

Fig. 22 depicts the embodiment of fig. 11 after the hypodermic needle has been put into the safe mode.

Fig. 23 is a partial view of a specially designed syringe with a long neck portion, to be used with some versions of some of the embodiments.

Fig. 24 is a side view of the eleventh embodiment of the invention.

No spring is used in this embodiment.

Fig. 25 shows the embodiment of fig. 24 after it has been put into the safe mode.

Fig. 26 shows a variation of the embodiments of figs. 24 and 25.

Fig. 27 shows another variation of the embodiments of figs. 24 and 25.

Fig. 28 is a side view of the twelfth embodiment of the invention.

Fig. 29 is a partial expanded view, from the side, of the embodiment of fig. 28, showing a variation of that embodiment.

Fig. 30 is another partial expanded view, from the side, of the embodiment of fig. 28, showing a variation of that embodiment.

Fig. 31 is a partial view, from the top, showing a variation of the embodiment of fig. 28.

Fig. 32 is a cross-sectional view of a hollow member, showing the hole through the hollow member, with the needle in the hole.

Fig. 33 is a cross-sectional view of another hollow member wherein the hole, or groove, is open to a side of the hollow member as well as the ends. The needle is shown inside.

Fig. 34 is a cross-sectional view of a hollow member of similar structure to that depicted in fig. 33. The needle is shown inside.

Fig. 35 is a side view of an embodiment similar to that shown in fig. 24, but with a spring added.

Fig. 36 is a side view of an embodiment similar to that shown in fig. 26, but with a spring added.

Fig. 37 is a side view of an embodiment similar to that shown in fig. 28 with the addition of a spring.

Fig. 38 shows an embodiment similar to that shown in fig. 15, but where the hub includes an encircling groove.

Fig. 39 shows an embodiment similar to that shown in fig. 16, but where the hub includes an encircling groove.

Fig. 40 is a perspective view of an embodiment of the invention that uses releasers to release the supporting members.

Fig. 41 is a perspective view of another embodiment of the invention that uses releasers.

Fig. 42 shows a slight variation of the end portion of the supporting member shown in fig. 41.

Fig. 43 shows another variation of the end portion of a supporting member, as might be used in the embodiments of figs. 40 or 41.

Fig. 44 is a side view of another embodiment of the invention in which releasers are used.

Fig. 45 is a side view of a further embodiment of the invention that uses releasers but not a spring.

Fig. 46 shows a side view of still another embodiment that utilizes releasers.

Fig. 47 is a side view showing yet another embodiment of the invention that utilizes releasers.

Fig. 48 is a side view depicting another embodiment of the invention that uses releasers but not a spring.

### DETAILED DESCRIPTION

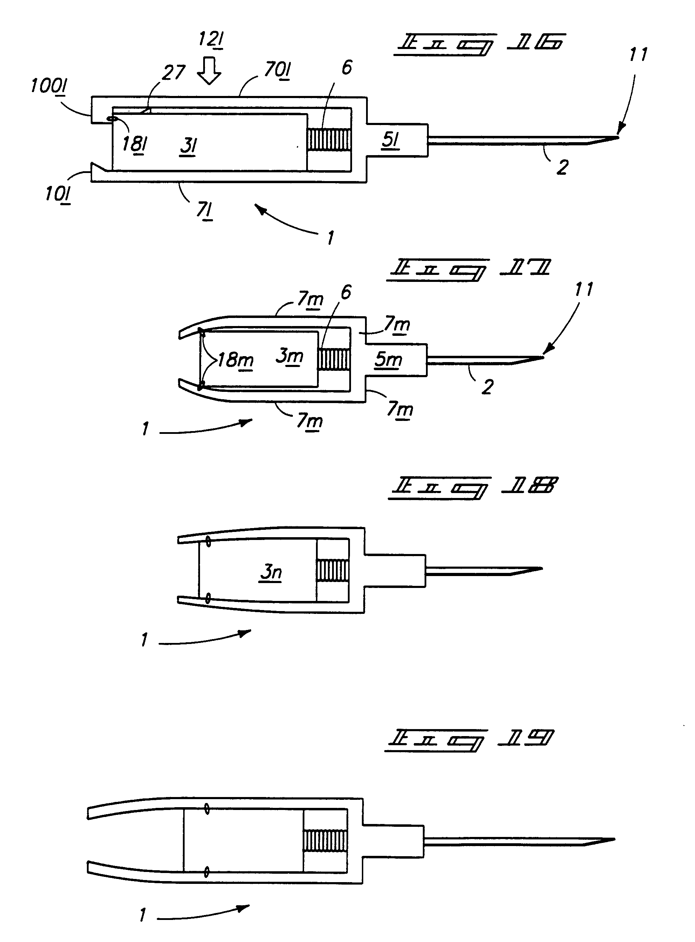

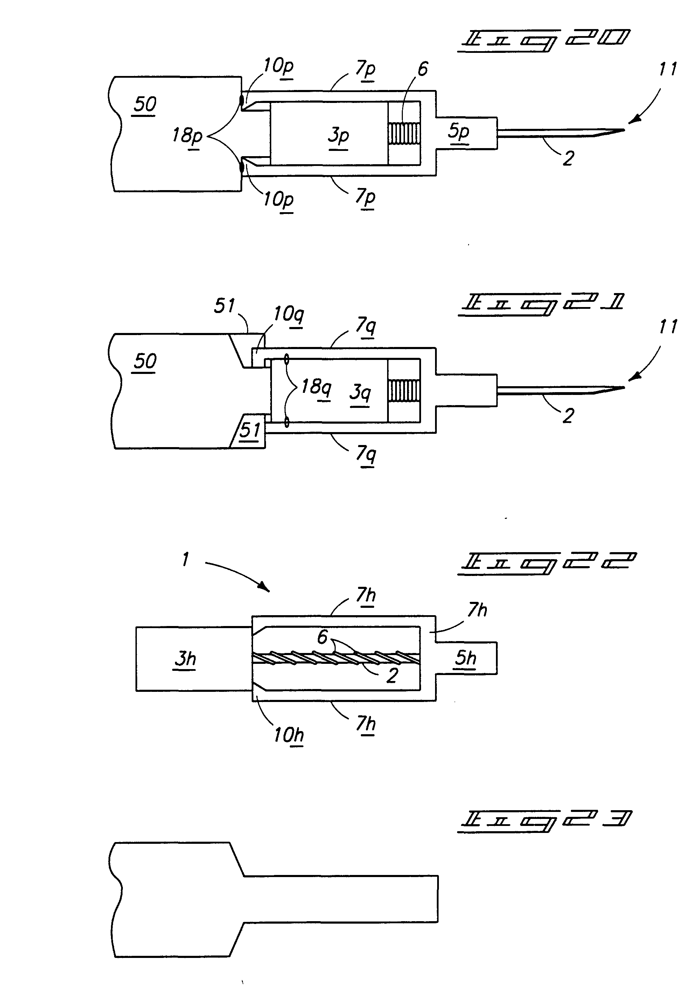

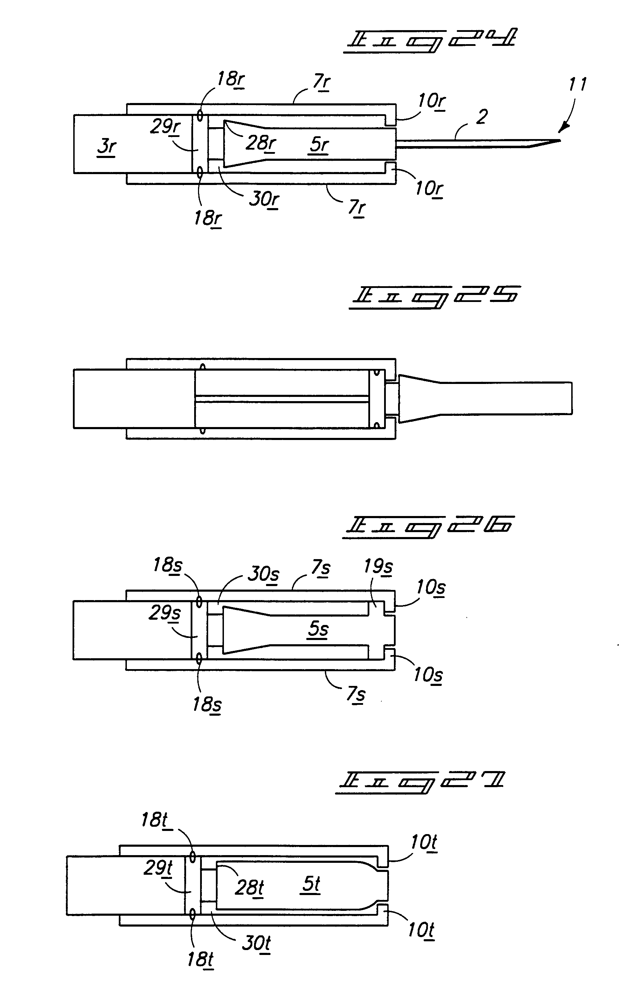

In all of the embodiments described herein, it is generally preferred that all parts other than the needles 2, springs 6, and the small pieces of spring steel 15 used in the third embodiment (figure 7) are formed of plastic. This preference, however, does not preclude the option of using materials other than plastic if such other materials prove advantageous to plastic either in the function of the invention, or in its manufacture.

All of the following embodiments include a hub, which is designated by the numeral 3 followed by a letter, and a hollow member, which is designated by the numeral 5 followed by a letter. Where the hubs of the various embodiments are formed of plastic as preferred, it is also preferred that all plastic parts connected directly with the plastic hubs of the various embodiments, or indirectly through a continuum of plastic, be formed with those hubs by a plastic molding process, such as injection molding. However, methods other than molding may be used to join these parts if desired. It is similarly preferred that, where the hollow members of the various embodiments are formed of plastic as preferred, all plastic parts connected directly with the hollow members, or indirectly through a continuum of plastic, be formed with those hollow members by a plastic molding process, such as injection molding. Again, however, methods other than molding may be used to join these parts if so desired.

Where the hubs of the various embodiments, are formed of plastic, the needles 2 may be attached to those plastic hubs either by being molded into the hubs in the plastic molding process as molded-in inserts, or they may be attached to the hubs after the hubs have been molded. In the latter case, they would preferably be inserted into a hole formed into the end of the hub and be secured to the hub by any of a variety of methods such as those which are described in the trade as cementation, ultrasonic installation, or press-in insertation, for example. In either case, it may be desired to have an intermediary material attached to the outside of the needle 2 where it is joined to the hub. If used, this intermediary material would function to increase the surface area of the junction with the plastic hub, thus increasing the strength of that junction, and it may also be a material selected to join more effectively with the plastic of the hub than would the material of which the needle 2 is composed. However, for the sake of keeping manufacturing costs to a minimum, it may be desirable not to use such an intermediary material.

Most of the following embodiments include a spring 6, and in most embodiments where a spring 6 is used, those springs 6 are connected at one end with the hub, and at the other end with the hollow member. Insofar as those hubs and/or hollow members are formed of plastic, the preferred methods of attachment of the spring 6 with the hub, and the spring 6 with the hollow member are the same as between needle 2 and hub; that is, being molded in as molded-in inserts, ultrasonic installation, and cementation. The method of attaching the spring 6 may, of course, vary between the hub and the hollow member. Where the hub and/or the hollow member are not formed of plastic, cementation may still be employed as a means of attaching the spring 6 to the hub and/or the hollow member.

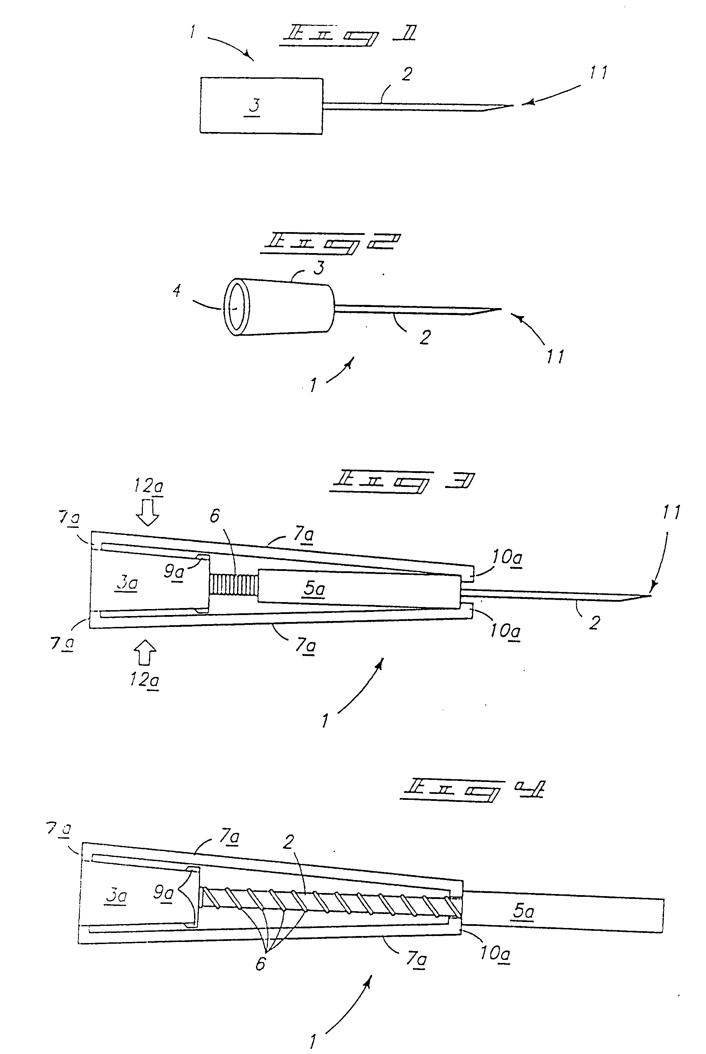

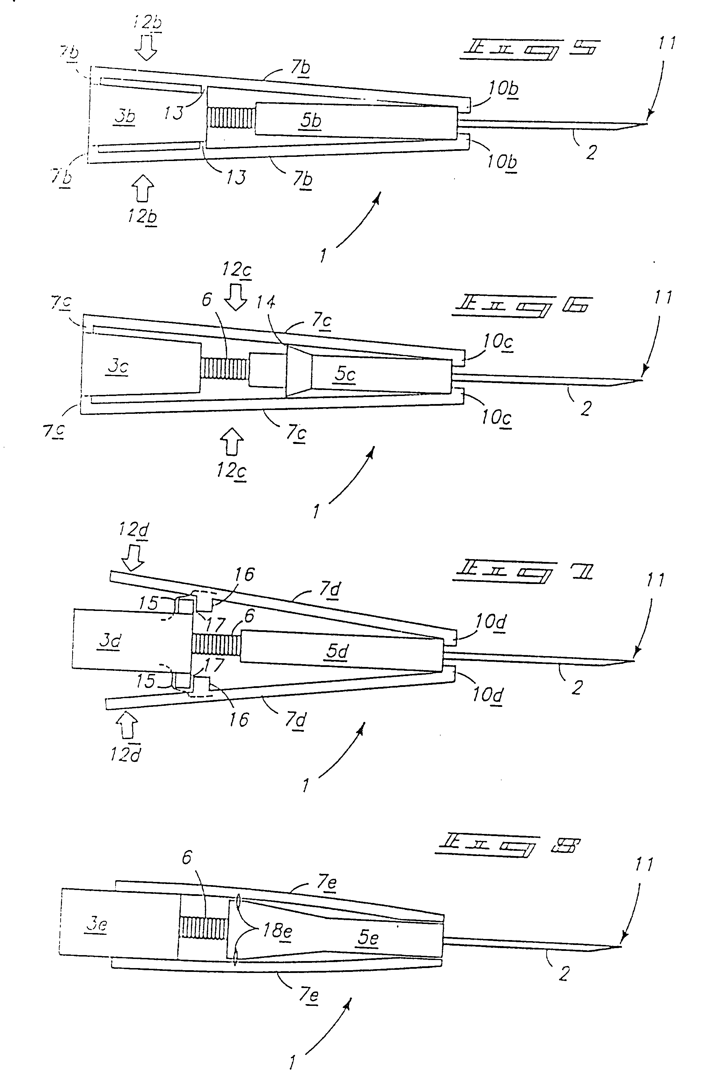

The conventional portion of the hypodermic needle 1 is composed of the needle 2 and the hub 3. Figures 1 and 2 depict a conventional hypodermic needle 1. As shown in figure 2, one end of the hub 3 includes an opening 4 which accepts the syringe or I.V. attachment.

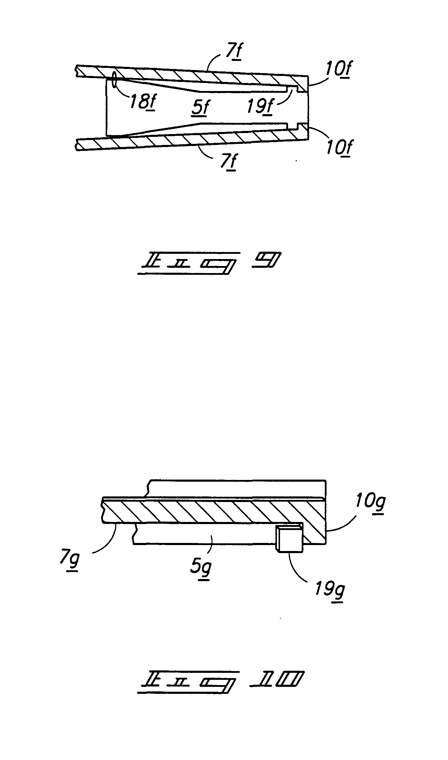

In the first embodiment of the present invention, illustrated in figure 3, a hollow member 5A is positioned over a length of the needle 2. A spring 6 is positioned between the hub 3A and the hollow member 5A, encircling the needle 2. The spring 6 is connected at one end to the hollow member 5A, and at the other end to the hub 3A. Two supporting members 7A extend from the rear of the hub 3A toward the piercing end 11 of the needle 2, each of the two supporting members 7A being located approximately 180° from the other around the hub 3A. At the forward end of the hub 3A two bumps 9A extend outward from the hub 3A, so that a bump 9A is located directly beneath each of the supporting members 7A; the bumps 9A are not connected to the supporting members 7A. At the forward end of each of the supporting members 7A a protrusion 10A extends inward from the supporting members 7A toward the needle 2. The protrusions 10A hold the hollow member 5A away from the piercing end 11 of the needle 2 against tension from the spring 6.

A number of the embodiments described throughout the detailed description utilize a protrusion, which is designated by the numeral 10 or 100 followed by one or two letters. As used and defined herein and in the appended claims, this protrusion is not a separate element but rather a part or feature of the supporting member (which is an element).

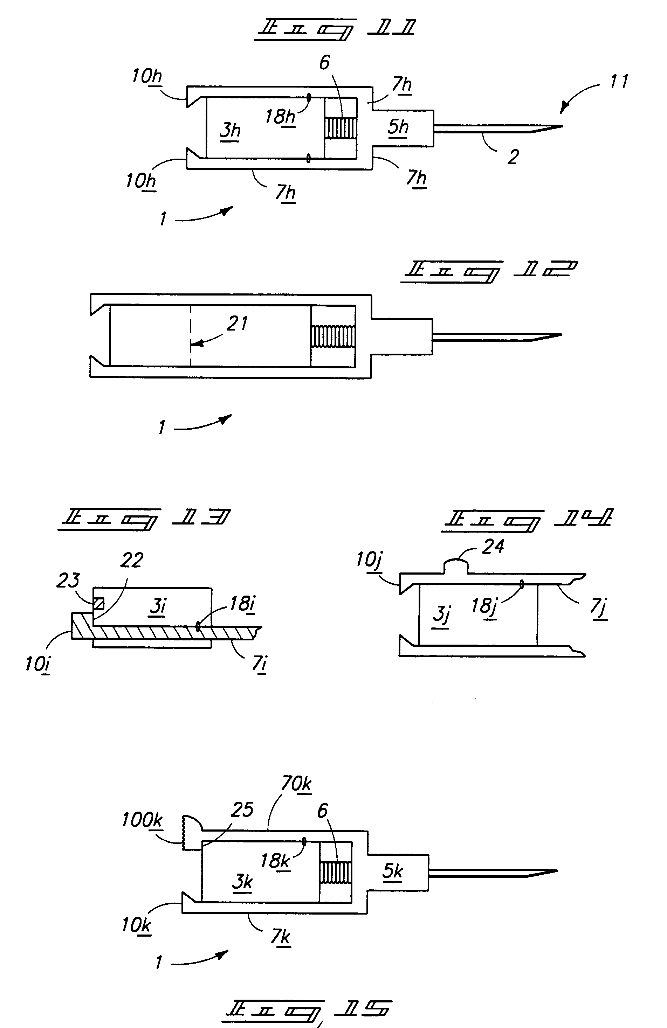

While in use, the hypodermic needle 1 is generally safe from contamination prior to its being inserted into the patient. It is after the needle has been removed that it is potentially hazardous to others. In practice, the person removing the hypodermic needle 1 of the first embodiment from the patient squeezes inwardly on the two supporting members 7A, preferably with his or her thumb and forefinger, just rearward of the bumps 9A at a position indicated generally by the arrows 12A. In response to this inward movement, the two bumps 9A act as fulcrums to the supporting members 7A, causing the forward end of the supporting members 7A and the protrusions 10A to move outward away from the needle 2. This movement releases the hollow member 5A, causing it to move forward by the force of the spring 6 to cover the piercing end 11 of the needle 2. After the hollow member 5A has passed the protrusions 10A, and finger pressure on the supporting members 7A has been released, the forward ends of the supporting members 7A move inward toward the needle 2 in order to retain their natural position. This places the protrusions 10A behind the hollow member 5A, and thereby prevents the hollow member 5A from being accidentally pushed rearward exposing the piercing end 11 of the needle 2, as illustrated in figure 4.

Figure 5 depicts a slight variation of the first embodiment in which the bumps 9A of figures 3 and 4 are replaced with forward bridges 13. The forward bridges 13 are connected to the hub 3B and the supporting members 7B, and are thinner than the bumps 9A of figures 3 and 4 when measured longitudinally of the hypodermic needle 1. When squeezing inwardly on the supporting members 7B at a position indicated generally by the arrows 12B of figure 5, the forward bridges 13 are thin enough to bend rearward while acting as fulcrums for the supporting members 7B. This enables the front of the supporting members 7B and the protrusions 10B to move away from the needle 2 and release the hollow member 5B.

In the second embodiment of the invention, depicted in figure 6, the construction is similar to the first embodiment as depicted in figures 3 and 4, except that the bumps 9A of figures 3 and 4 are absent and their function is replaced by the ridge 14 located on the hollow member 5C (figure 6). When inward pressure is applied to the supporting members 7C, as with thumb and forefinger, at a position indicated generally by the arrows 12C, the ridge 14 acts as the fulcrum to the supporting members 7C, causing the forward end of the supporting members 7C and the protrusions 10C to move outward away from the needle 2. As a result, the hollow member 5C is released by the protrusions 10C and moves forward by the force of the spring 6. The inclined forward edge of the ridge 14 allows the ridge 14 to pass by the protrusions 10C. After the hollow member 5C has covered the piercing end 11 of the needle 2, the protrusions 10C lodge behind the hollow member 5C and thereby prevent the hollow member 5C from being accidentally pushed rearward, exposing the piercing end 11 of the needle 2. The advantage of the second embodiment over the first is the greater force of leverage transmitted to the outward movement of the protrusions, making it easier to actuate the release of the hollow member. Also, since the distance along the supporting members between the fulcrum and the protrusions is shorter in the second embodiment than it is in the first, that segment of the supporting members would be more rigid and less likely to bend adversely in response to the frictional resistance between the protrusions and the hollow member resulting from the pressure from the spring. Such bending could prevent the release of the hollow member.

In the third embodiment depicted in figure 7, each of the two supporting members 7D are connected to the hub 3D with a separate piece of spring steel 15 formed from a flat elongate piece of stock. The shape of these pieces of spring steel 15 and the manner in which they connect the hub 3D and with two supporting members 7D cause the ends of the two supporting members 7D to push inward toward the needle 2. When finger pressure is applied inwardly on the end portions of the two supporting members 7D, at a location indicated generally by the arrows 12D, the two pieces of spring steel 15 act as fulcrums to the two supporting members 7D, causing the ends of the supporting members 7D and the protrusions 10D to move away from the needle 2 thus releasing the hollow member 5D to move forward by the force of the spring 6. As with the previous embodiments, after the hollow member 5D has covered the piercing end 11 of the needle 2, the protrusions 10D move inward, toward the needle 2 by the force of the piece of spring steel 15, and lodge behind the hollow member 5D. With the protrusions 10D behind the hollow member 5D, and the protuberances 16 on the supporting members 7D aligned with the protuberances 17 on the hub 3D, the hollow member 5D is prevented from being pushed rearward exposing the piercing end 11 of the needle 2.

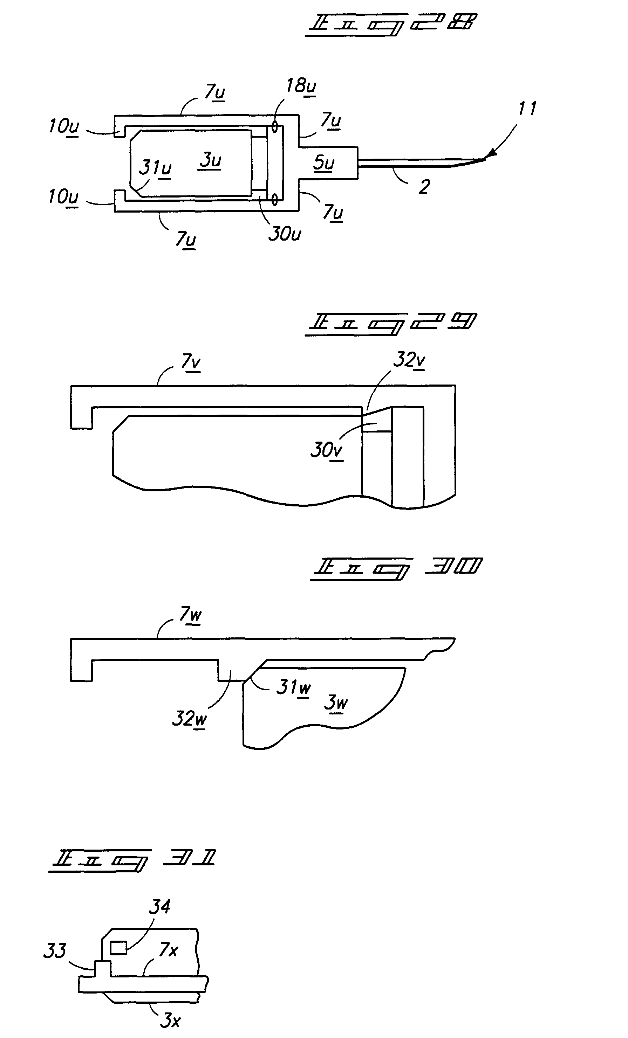

In the fourth embodiment shown in figure 8, two supporting members 7E extend forward from the hub 3E, curving inward toward their forward ends. The two supporting members 7E are connected with the hollow member 5E by the seals 18E which releasably connect the supporting members 7E with the hollow member 5E at an isolated spot where the two are in contact, as indicated by the reference character 18E. These seals 18E, which are employed on several of the embodiments described herein and are denoted by the numeral 18 followed by one or two letters, are formed in any of a variety of ways, including but not limited to: The addition of a small amount of melted plastic (from another source) to releasably join the two or more components; the addition of another type of adhesive (as glue or cement) to releasably join the components, and, if both components to be releasably joined are formed of plastic, melting a small amount of plastic from each of the two components together at an isolated spot where the two components are in contact, or nearly in contact, so that the melted plastic joins and solidifies together. This last mentioned type of seal could be formed by touching the components to be releasably joined with a hot piece of metal or other material. However formed, the seal is broken as the user is putting the hypodermic needle in the safe mode.

The seals 18E between the supporting members 7E and the hollow member 5E hold the hollow member 5E away from the piercing end 11 of the needle 2 against the tension of the spring 6. In practice, once the needle 2 has been removed from the patient, the person removing the needle 2 may perform one of several actions in order to break the seals 18E. In one such action the hollow member 5E is grasp, as with thumb and forefinger, and twisted relative to the supporting members 7E. Another action calls for pushing forward on the rear of the hollow member 5E, preferably with either thumb or forefinger. In still another action, the user may squeeze inwardly on the supporting member 7E, preferably with thumb and forefinger, as at a position between the rear of the hollow member 5E and the front of the hub 3E, behind the seals 18E. A similar squeezing action on the supporting members 7E forward of the seals 18E may also break the seals 18E. Once the seals 18E have been broken, the hollow member 5E moves forward by the force of the spring 6. The outside of the hollow member 5E flares outward, increasing in diameter toward the rear of the hollow member 5E. This flared shape allows the hollow member 5E to move past the ends of the supporting members 7E, bending the supporting members 7E outward slightly as the hollow member 5E moves forward. Once the hollow member 5E has passed the ends of the supporting members 7E and has covered the piercing end 11 of the needle 2, the ends of the supporting members 7E move back inward toward the needle 2 and lodge behind the rear surface of the hollow member 5E, preventing accidental rearward movement of the hollow member 5E. Since the supporting members 7E curve toward each other toward their fronts, the supporting members 7E are closest together at their front ends. The distance between the supporting members 7E at their front ends is less than the diameter of the rear surface of the hollow member 5E, though, the distance between the two supporting members 7E at the hub 3E is greater than the diameter of the rear surface of the hollow member 5E.

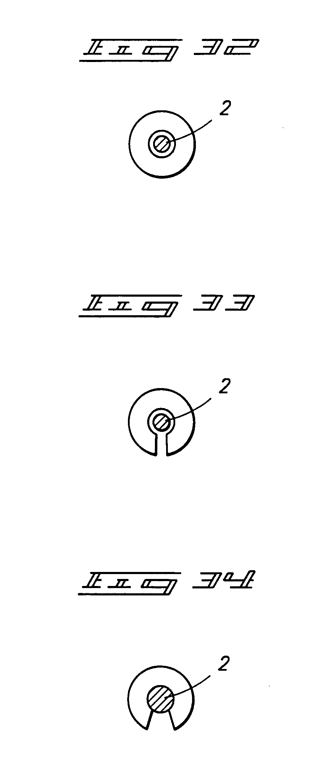

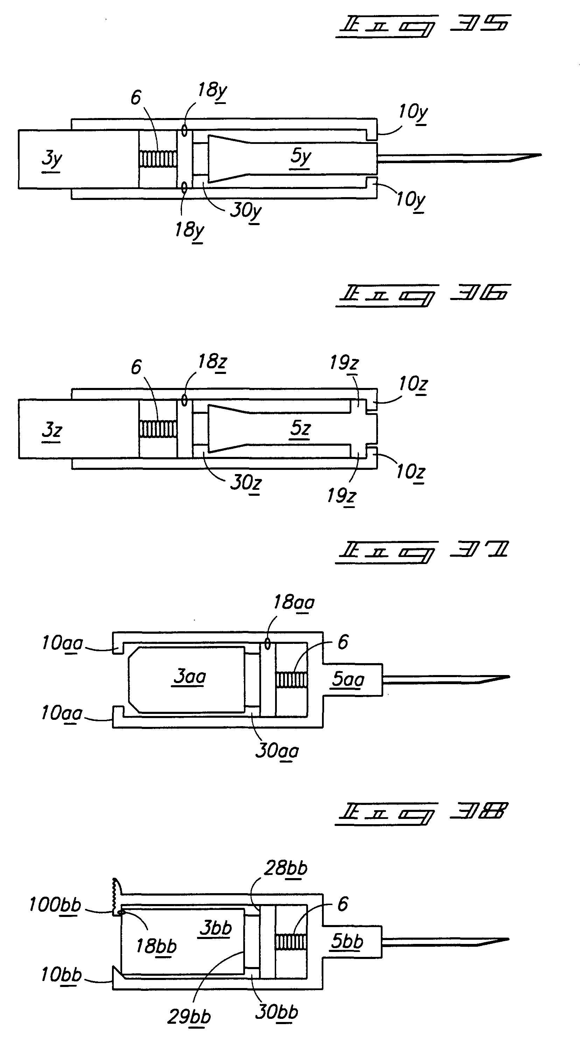

Figures 9 and 10 show variations of the fourth embodiment in which securement means in addition to the seals are used to hold the hollow member 5F against tension from the spring. Figure 9 is a side view showing that portion of the embodiment where the variation exists. In the variation depicted in figure 9, a protrusion 10F extends inward from the end of each supporting member 7F. A projection 19F extends outward from the hollow member 5F behind each protrusion 10F. Twisting of the hollow member 5F relative to the supporting members 7F releases the protrusions 10F from the projections 19F, as well as breaking the seals 18F. Only one seal 18F is shown in figure 9 to indicate that the other seal is located on the other side of the lower supporting member 7F. This allows for smoother, unobstructed forward movement of the hollow member 5F when the hollow member 5F is twisted in a certain direction. Such positioning of the seals is an option on all embodiments where two supporting members are attached with seals.

Figure 10 is a top view that also shows that part of the embodiment where the variation exists. In the variation depicted in figure 10, a side protrusion 10G extends laterally from the end portion of each supporting member 7G. A projection 19G, similar or identical to the projection 19F shown in figure 9 extends outward from the hollow member 5G behind each side protrusion 10G. In a manner similar to the variation depicted in figure 9, twisting of the hollow member 5G relative to the supporting member 7G releases the side protrusions 10G from the projections 19G as well as breaking the seals.

The manner in which the first version of the fourth embodiment (figure 8) is designed would enable the hub 3E and supporting members 7E to be formed together by a plastic molding process, such as injection molding, in a mold that, in terminology of the trade, would not require the use of slides. This should be the case either if the hub 3E and supporting members 7E are molded without the needle 2, or if the needle 2 is molded into the hub 3E as a molded-in insert. The necessity of using a mold with slides increases production costs for the item molded.

One disadvantage of the embodiments described thus far is that a large proportion of the needle 2 segment of the hypodermic needle 1 must be covered with the hollow member and spring 6 prior to and during its use. The segment of the needle 2 thus covered cannot be utilized for its intended purpose. Because of this, the hypodermic needle 1 must be constructed with a longer needle 2 segment than would otherwise be needed for any particular application.

In the following embodiments, the proportion of the needle 2 segment of the hypodermic needle 1 covered by the hollow member is kept to a minimum by a basic design change from those previous embodiments. This design change, however, does necessitate the lengthening of the hub portion of the hypodermic needle 1, except when the desired needle 2 length is relatively short, or when a specially designed syringe with a long neck is used with the hypodermic needle 1. Such a specially designed syringe is depicted, in part, in figure 23. Whether or not the hub must be lengthened to accommodate this design, it would likely be more economical to increase the length of the hub, especially if the hub is formed of plastic, than it would be to increase the length of the steel needle 2 segment of the hypodermic needle 1.

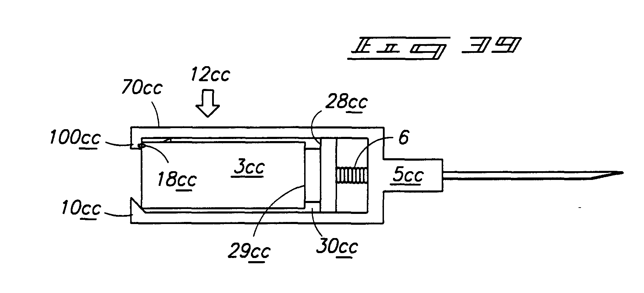

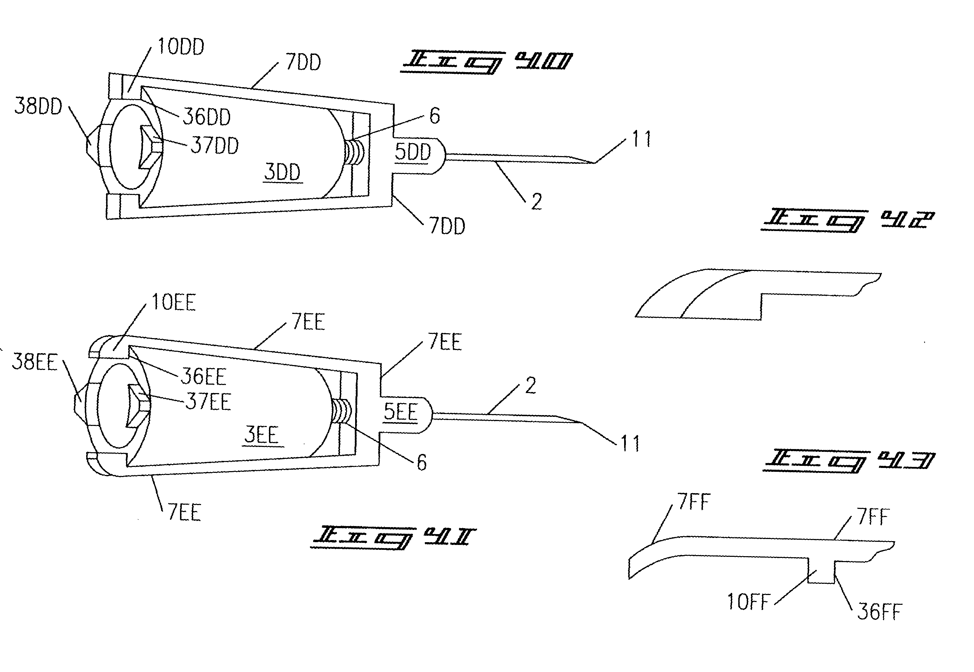

In the several following embodiments, the two supporting members are attached to the hollow member rather than the hub. In the fifth embodiment of the invention depicted in figure 11, two supporting members 7H are connected with the hollow member 5H and extend rearward from the hollow member 5H. A protrusion 10H is located at the rear of each supporting member 7H. The supporting members 7H are connected to the hub 3H with seals 18H, which hold the hollow member 5H away from the piercing end 11 of the needle 2 against tension from the spring 6. The hollow member 5H is released by a twist of the supporting members 7H, preferably with thumb and forefinger, in a short circular motion around the outside of the hub 3H, which breaks the seals 18H. The inclined forward portion of the protrusions 10H allow the protrusions 10H to move past the end of the hub 3H as the hollow member 5H moves forward by the force of the spring 6. After the hollow member 5H has covered the piercing end 11 of the needle 2, the protrusions 10H lodge in front of the hub 3H. This prevents accidental rearward movement of the hollow member 5H exposing the piercing end 11 of the needle 2, as shown in figure 22. Figure 12 depicts another example of this same embodiment where a longer needle segment is used, necessitating a longer hub. The dashed line 21 indicates the rear border of a shorter hub that could be used if the hypodermic needle is used with a specially designed syringe with a long neck, as the syringe depicted, in part, in figure 23.

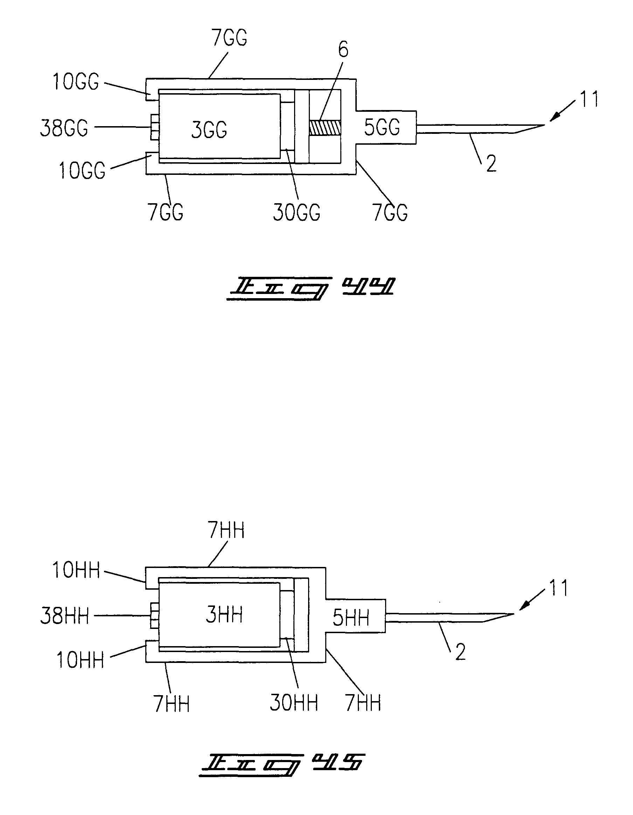

Figure 13 shows a top view of the hub section in a slight variation of the fifth embodiment. The variation involves widening the rear portion of the supporting members 7I, including the protrusions 10I, to form a step 22. Two projections 23 extend outward at the rear portion of the hub 3I, each projection 23 being aligned with the step 22 on one of the supporting members 7I. The steps 22 with the projections 23 provide additional securement, along with the seals 18I, in holding the hollow member rearward against tension of the spring. The same twisting motion of the supporting members 7I described initially for the fifth embodiment releases the steps 22 from the projections 23, as well as breaking the seals 18I to release the hollow member. Of course, with this variation, one or more of the seals 18I could be eliminated, or the projection 23 and step 22 may be utilized on only one of the supporting members 7I. Figure 13 shows the hub as it would appear after the step 22 on the supporting members 7I has been twisted free of the projection 23.

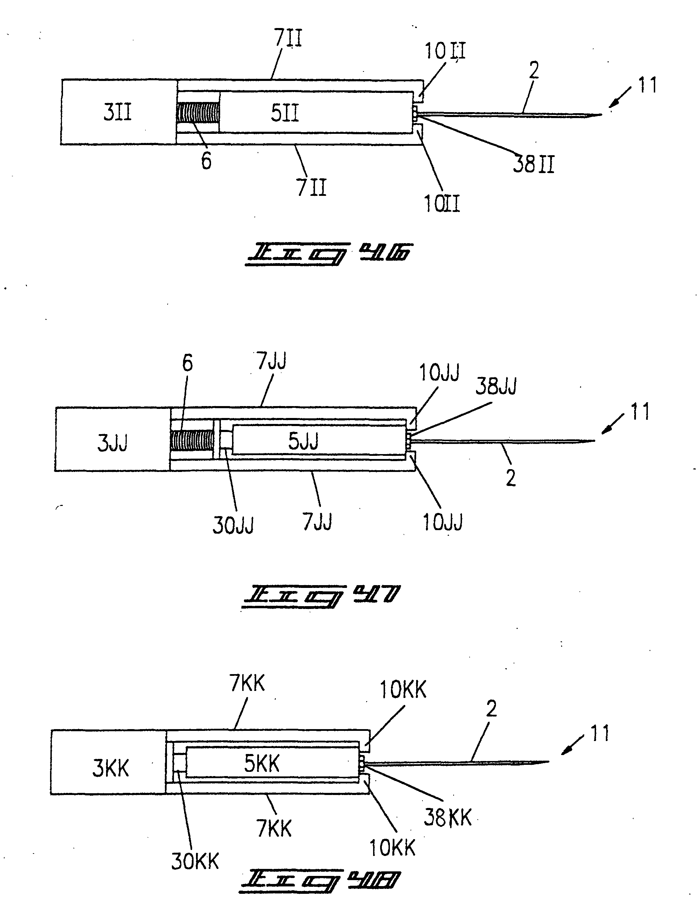

Figure 14 shows the hub section of another variation of the fifth embodiment. In this example, only one of the two supporting members 7J is secured to the hub 3J with a seal 18J. That same supporting member 7J includes a knob 24 on its top. Release of the hollow member is actuated by pushing forward on the knob 24 as with thumb or forefinger, and thus breaking the seal 18J.

In the sixth embodiment, depicted in figure 15, a flat front surface 25 on one of the protrusion 100K rests against the rear of the hub 3K. This helps support the hollow member 5K against the tension of the spring 6. The same supporting member 70K on which the protrusion 100K is located is attached to the hub 3K with a seal 18K. The other supporting member 7K is not attached to the hub 3K. Thumb or finger pressure on the rear of the protrusion 100K, pushing upward and forward, releases the protrusion 100K from the hub 3K allowing the hollow member 5K to spring forward. Both protrusions 100K and 10K align with the front of the hub 3K to prevent accidental rearward movement of the hollow member 5K.

In the seventh embodiment depicted in figure 16, a flat front surface on one of the protrusions 100L rests against the rear of the hub 3L. A ridge 27 extends outward from the hub 3L directly underneath the supporting member 70L. The supporting member 70L is held away from the hub 3L except at the ridge 27. A seal 18L connects the protrusion 100L with the hub 3L. The seal may alternately be located between the supporting member 70L and the ridge 27, or may be eliminated altogether. The other supporting member 7L is not sealed to the hub 3L. To release the hollow member 5L, a person pushes inward and forward on the supporting member 70L, at a position forward of the ridge 27, at a location indicated generally by the arrow 12L. The inward movement of the supporting member 70L at this location causes the protrusion 100L to move outward in relation to the hub 3L, and together with the forward movement causes the protrusion 100L to be released from the hub 3L. The seal 18L is broken in the process, and the hollow member 5L moves forward to cover the piercing end 11 of the needle 2. After lodging in front of the hub 3L, the protrusions 100L and 10L prevent rearward movement of the hollow member 5L.

The eighth embodiment is shown in figures 17 and 19, wherein figure 19 depicts a version with a longer needle to be used with a syringe with a long neck, such as the syringe shown, in part, in figure 23. In this embodiment the supporting members 7M curve inward toward the rear so that the supporting members 7M are closest together at their ends. The supporting members 7M are connected with the hollow member 5M, and are connected to the hub 3M with the seals 18M. A short twisting of the supporting members 7M around the periphery of the hub 3M (as in the fifth embodiment) causes the seals 18M to break. Another action by which the seals 18M may be broken is squeezing inwardly on the supporting members 7M, preferably with thumb and forefinger, as at a location just forward of the seals 18M, or, alternately, just rearward of the seals 18M. Once broken, the hollow member 5M travels forward by the force of the spring 6, bending the end portions of the supporting members 7M outward slightly as they pass the hub 3M. Since the distance between the ends of the supporting members 7M is less than the diameter of the front surface of the hub 3M, the ends of the supporting members 7M lodge in front of the hub 3M after the hollow member 5M has covered the piercing end 11 of the needle 2, preventing rearward movement of the hollow member 5M.

Figure 18 depicts a slight variation of this embodiment in which the hub 3N is formed to flare outward toward the front, so that the diameter of the front surface of the hub 3N is greater than the diameter of the rear of the hub 3N.

The manner in which the eighth embodiment is designed would enable the hollow member 5M and the supporting members 7M to be formed together by a plastic molding process, such as injection molding, in a mold that would not require the use of slides.

Occasionally, a hypodermic needle and a syringe are packaged as a single unit, such as to dispense a specified quantity of medicine. In the ninth embodiment, depicted in figure 20, a hypodermic needle/syringe combination is shown in which the two function as a single disposable unit to carry out the primary object of this invention. Seals 18P attach the protrusions 10P to the syringe 50 to hold the hollow member 5P against tension from the spring 6. A twist of the syringe 50 with one hand while holding the hub 3P of the hypodermic needle with thumb and forefinger of the other hand, breaks the seals 18P allowing the hollow member 5P to move forward. The presence of the thumb and forefinger on the hub 3P prevents the supporting members 7P from simply turning with the syringe 50, rather than breaking. The relatively wide syringe 50 offers a sturdy grip for the user to twist.

Although the hypodermic needle shown in this embodiment is essentially identical to the one depicted in figure 11, any of those previous embodiments in which the hollow member is released by twisting the supporting members may be used in combination with the syringe in the manner just described and shown in figure 20.

In the tenth embodiment as depicted in figure 21, a specially designed syringe is used with a separate hypodermic needle. As was the case for the embodiment depicted in figure 20, the hypodermic needle used in this example is essentially identical to the one depicted in figure 11, though any of those previously described hypodermic needles in which the hollow member is released by twisting the supporting members may be used with this type of syringe. Flaps 51 extending forward on the syringe 50 match with the ends of the supporting members 7Q and protrusions 10Q of the hypodermic needle. A twist of the syringe 50 while thumb and forefinger grasp the hub 3Q causes the flaps 51 to rotate the supporting members 7Q, thus breaking the seals 18Q. The flaps 51 of this syringe 50 may be combined with a long neck, as on the syringe depicted in figure 23, to be used with those hypodermic needles designed to be used with long neck syringes. In such a combination, it would likely be desirable to extend the flaps 51 of figure 21 further forward as well.

In the eleventh and twelfth embodiments, depicted in figures 24 through 31, a hollow member similar to those employed in the previous embodiments is pushed forward by the user, rather than by a spring. In the eleventh embodiment, depicted in figure 24, a front ridge 28R and a rear ridge 29R encircle the hollow member 5R to form an encircling groove 30R. Seals 18R connect the rear ridge 29R with the two supporting members 7R that extend forward from the hub 3R, and a protrusion 10R extends inward from the end of each supporting member 7R. In practice, the user breaks the seals 18R either by twisting the hollow member 5R relative to the supporting members 7R, or simply by pushing the hollow member 5R forward. After the seals 18R have been broken, the user pushes, or continues to push, the hollow member 5R forward toward the piercing end 11 of the needle 2. The inclined forward surface of the front ridge 28R allows the hollow member 5R to pass by the protrusions 10R, bending the supporting members 7R outward slightly, until the groove 30R reaches the protrusions 10R, whereupon the supporting members 7R spring inward pushing the protrusions 10R into the groove 30R. Once the protrusions 10R are positioned in the groove 30R the hollow member 5R is prevented from moving rearward exposing the piercing end 11 of the needle 2, or from moving forward off the end of the needle 2, as shown in figure 25.

In a variation of the eleventh embodiment depicted in figure 26, a projection 19S extends outward from the hollow member 5S behind each protrusion 10S to help prevent accidental breakage of the seals. Twisting of the hollow member 5S relative to the supporting members 7S breaks the seals 18S and releases the projections 19S from the protrusions 10S, allowing the hollow member 5S to be pushed forward.

Figure 27 depicts another variation of the eleventh embodiment in which the front ridge 28T extends to the rear of the protrusions 10T. In this variation, the front ridge 28T is very wide and is flat on its outer surface, and is convex just behind the protrusions 10T. In the variation depicted in figure 27, the seals 18T may, if desired, be eliminated altogether.

Figure 28 depicts the twelfth embodiment in which supporting members 7U extend rearward from the hollow member 5U. A protrusion 10U extends inward from the end of each supporting member 7U. The outside rear edge of the hub 3U is inclined 31U to allow passage of the protrusions 10U. A groove 30U encircles the hub 3U near the front of the hub 3U, and seals 18U temporarily connect the supporting members 7U with the hub 3U. In this example, the portion of the hub 3U located forward of the groove 30U extends outward slightly more than the portion of the hub 3U located behind the groove 30U. Twisting or pushing of the supporting members 7U or hollow member 5U breaks the seals 18U, allowing the hollow member 5U to be pushed forward to cover the piercing end 11 of the needle 2. As forward movement proceeds,. the supporting members 7U bend outward as the protrusions 10U move up the inclined 31U rear outside edge of the hub 3U, and subsequently spring inward as the protrusions 10U reach the groove 30U. Once in the groove 30U, the protrusions 10U prevent forward and rearward movement of the hollow member 5U, which now covers the piercing end 11 of the needle 2.

The twelfth embodiment may be adapted for a longer needle 2 by lengthening the hub 3U and supporting members 7U, or by lengthening only the supporting members 7U whereupon it would be used with a long neck syringe, as the syringe depicted in figure 23. The seals 18U may, if desired, be eliminated in this embodiment in those versions where the protrusions 10U rest directly or nearly directly behind the inclined 31U rear outside edge of the hub 3U. The seals 18U may also be eliminated, if desired, in the variations of this embodiment depicted, in part, in figures 29 and 30. In figure 29, a protuberance 32V extends inward from one or both supporting members 7V into the groove 30V. The inclined front of the protuberance 32V allows the protuberance 32V to be pushed out of the groove 30V. In figure 30, the protuberance 32W extends inward from one or both supporting members 7W directly behind the inclined 31W rear outside edge of the hub 3W. The example as depicted in figure 30 is designed for use with a long neck syringe, as the syringe in figure 23. The inclined front of the protuberance 32W allows the protuberance 32W to be pushed up the inclined 31W outside rear edge of the hub 3W, and past the groove (not shown).

Figure 31 depicts a top view of another variation of the twelfth embodiment in which a side protrusion 33 extends laterally from each supporting member 7X. A projection 34 extends outward from the hub 3X in front of each side protrusion 33. Alignment of the side protrusions 33 and the projections 34 helps to prevent accidental breakage of the seals. Twisting of the supporting members 7X relative to the hub 3X breaks the seals and releases the side protrusions 33 from the projections 34, allowing the hollow member to be pushed forward. The presence of the side protrusions 33 and the projections 34 allow the option of eliminating the seals.

In the eleventh and twelfth embodiments, and in the variations of the eleventh and twelfth embodiments, springs may be added between the hubs and the hollow members in order that those embodiments may function in the same manner as the first through tenth embodiments, in that the hollow members, once released, would move forward by the force of such springs rather than by being pushed by the user. Of course, the exact structure of those embodiments as shown in the figures would generally be modified in order to accommodate the presence of the spring. For example, the supporting member may be lengthened or the hollow member shortened, relative to the other. The springs may or may not be attached to the hubs and/or hollow members since, once in the groove (the groove designated by 30 followed by a letter), the protrusions (10 followed by a letter) would prevent forward as well as rearward movement of the hollow member, thus preventing the hollow member from sliding off the end of the needle 2. The fact that the spring need not necessarily be attached to the hollow member and/or the hub may constitute an advantage over those previous embodiments where springs were used, that advantage being a possible lower cost of manufacture. When used with springs, the twelfth embodiment as depicted in figure 28 may be used in combination with the syringe of figure 20 (without the seals 18U), and with the syringe of figure 21. The variation of the twelfth embodiment as depicted in figure 31 may also be used in combination with the syringes of figures 20 and 21.

Fig. 35 depicts an embodiment similar to that shown in fig. 24 but where a spring 6 is used to push the hollow member 5Y forward once the seals 18Y are broken. Positioning of the protrusions 10Y in the groove 30Y prevents rearward or forward movement of the hollow member 5Y.

Fig. 36 shows an embodiment similar to that shown in Fig. 26 but with a spring 6 added. In addition to the positioning of the protrusions 10Z in front of the projections 19Z, the seal 18Z helps prevent accidental release of the hollow member 5Z as well as indicating that the needle has not been used. Again, once the protrusions 10Z lodge in the groove 30Z the hollow member 5Z is prevented from moving rearward or forward.

Fig. 37 depicts the embodiment of fig. 28 but where a spring 6 pushes the hollow member 5AA forward once the seal 18AA has been broken. Positioning of the protrusions 10AA in the groove 30AA prevents rearward or forward movement of the hollow member 5AA. Variations to this embodiment (with a spring) include those depicted in Figs. 29, 30 and 31.

In the embodiments depicted in Figs. 35, 36 and 37 the spring 6 need not (if desired) be attached to either the hub or hollow member.

It will also be noted that the hollow member of figures 24, 26 and 27 may be employed in the first, second, and third embodiments (figures 3, 4, 5, 6 and 7), or with the first variation of the fourth embodiment (figure 9), in place of the hollow members used in those embodiments. When so employed, the protrusions of those first 3 embodiments (10A, 10B, 10C, and 10D including the variation) or the protrusion 10F of figure 9 would hold the hollow member away from the piercing end of the needle by lodging in front of the hollow member 5R of figure 24, in front of the projections 19S of figure 26, or in front of the convex forward edge of the (wide) front ridge 28T on the hollow member 5T of figure 27. Seals may or may not be used in these combinations, depending on the particular application and preference. After the hollow member has been released, those protrusions 10A, 10B, 10C, 10D, or 10F would lodge in the groove 30R, 30S, or 30T of figures 24, 26, and 27 respectfully, preventing forward as well as rearward movement of the hollow member. When the hollow members 5R, 5S, or 5T of figures 24, 26, or 27 respectfully are used with any of the first four embodiments, in place of those hollow members described for use with those embodiments, the spring 6 may or may not be attached to the hub and/or hollow member. The spring 6 may also be eliminated altogether, though use without the spring 6 is best suited for the variation of the fourth embodiment (figure 8) depicted in figure 9. When used without a spring, seals may or may not be used, again, depending on the particular application and preference. When the hollow members 5R, 5S, or 5T are used in combination with the second embodiment (figure 6), the rear ridge 29R, 29S, or 29T of the hollow members 5R, 5S, and 5T respectfully would act as the fulcrum to the supporting members 7C of figure 6 in place of the ridge 14 as initially described for that function in that embodiment.

Also, the sixth and seventh embodiments (figures 15 and 16 respectfully) may employ a hub with a groove, as the groove 30U depicted in figure 28, though such a groove would have to be wide enough to accommodate the protrusion 100k of figure 15, or the protrusion 100L of figure 16. With the protrusion 100K of figure 15, or the protrusion 100L of figure 16 lodged in a groove in the hub as the groove 30U of figure 28, the hollow member would be prevented from moving rearward or forward. In this combination, a spring as the spring 6 may or may not be used, and if used, the spring may or may not be attached to the hub and/or hollow member. The use of seals is also optional in these combinations, both with and without the use of a spring.

Fig. 38 depicts an embodiment similar to that shown in Fig. 15 but where the hub 3BB includes an encircling groove 30BB. As is the case with the embodiments of Figs. 37 and 39, the ridge 28BB in front of the groove 30BB is higher than the ridge 29BB in the rear of the groove 30BB. Once the protrusion 100BB has been lifted over the rear of the hub 3BB, and the seal 18BB has been broken, the spring 6 pushes the hollow member 5BB forward until the protrusion 100BB lodges in the groove 30BB, thus preventing rearward or forward movement of the hollow member 5BB. The other protrusion 10BB also helps prevent rearward movement of the hollow member 5BB. In this embodiment, the spring 6 need not be attached to either the hub 3BB or hollow member 5BB.

Fig. 39 depicts an embodiment similar to that shown in fig. 16 but where the hub 3CC includes an encircling groove 30CC. Pushing downward on the supporting member 70CC, as at 12CC, causes the protrusion 100CC to move upward over the rear of the hub 3CC, while breaking the seal 18CC in the process. The spring 6 pushes the hollow member 5CC forward until the protrusion 100CC lodges in the groove 30CC, thus preventing forward or rearward movement of the hollow member 5CC. The other protrusion 10CC aids in preventing rearward movement of the hollow member 5CC. Again, the spring 6 need not be attached to either the hub 3CC or the hollow member 5CC.

In another combination of features used in the various embodiments, it will be noted that the knob 24 of figure 14 may be used in combination with the twelfth embodiment depicted in figure 28, and the variations of the twelfth embodiment depicted in figures 29 and 30. The knob 24 of figure 14 may also be used in the seventh embodiment depicted in figure 16, the knob 24 being located on the supporting member 70L at a location indicated generally by the arrow 12L. Of course, the knob 24, or a similar feature may be used to increase one's grip on a supporting member of any embodiment where one or more supporting members are released by pushing or twisting.

In a number of the proceeding embodiments a seal (as denoted by the numeral 18 followed by one or two letters) was described to form a breakable connection between components. As explained earlier, the seal 18 can be formed in any of a variety of ways and is broken as the user is putting the hypodermic needle in the safe mode. As described thus far, the seal 18 has been used primarily to hold the hollow member away from the piercing end of the needle (against tension from the spring where applicable), and to prevent accidental release of the hollow member where another means is also used to hold the hollow member away from the piercing end of the needle. Another function of the seal 18 is to assure users that, if unbroken, the hypodermic needle has probably not been used, and, if broken, the hypodermic needle is likely contaminated even though the hollow member may have been re-positioned away from the piercing end of the needle. For these latter two functions, (indicator of contamination and secondary holding means), the seal 18 may be included on those embodiments previously described without having a seal 18. For example, a seal may be used in the first embodiment (figs. 3 and 4) between the bumps 9A and the supporting members 7A, or between the protrusions 10A or supporting members 7A and the hollow member 5A; in the second embodiment (fig. 6) between the supporting members 7C and the ridge 14, or between the protrusions 10C or ends of the supporting members 7C and the hollow member 5C; and in the third embodiment (fig. 7) between the protuberances 16 and the protuberances 17, or between the protrusions 10D or the ends of the supporting members 7D and the hollow member 5D.

In all embodiments where a seal 18 is or can be used, the function of the seal can include that of indicating use or non use and/or to act as a secondary securement means (where another means is also employed) for holding the hollow member away from the piercing end of the needle, whether or not such functions are mentioned in the descriptions of those embodiments.

Where the seal 18 has been described to hold one component to another, it may be used on both sides of that component. For example, in the various embodiments where a supporting member has been described as being attached to a hub or hollow member with a seal 18, the supporting member may be attached to that hub or hollow member with seals 18 on both sides of the supporting member. Also, where two identical or similar components, such as supporting members, have been described as being attached to another component with a seal 18, only one of those two may be so attached, if desired, in order to make it easier to break free. Finally, the seal 18 itself may be replaced with a feature of similar function or otherwise be formed in a manner not previously described. Especially in those cases where the purpose of the seal is only to indicate whether or not prior use has taken place and/or to act as a secondary securement means where another means is also employed to hold the hollow member away from the piercing end of the needle, a seal may be formed of paper or plastic (or like material) with adhesive applied to one surface thereof and joined to two separate components that move relative to each other when being placed in the safe mode. If such a seal has been torn to form two pieces, prior use is indicated as likely. Of course, such a seal could be used as the primary or only means of holding the hollow member away from the piercing end of the needle. Furthermore, any of the other various examples of seals described above could be used where the purpose is only to indicate prior use or non use.

Although all embodiments herein described utilize two supporting members, all of these embodiments, and variations thereof, are adaptable to be used with only one such supporting member. besides lowering manufacturing costs, the use of only one supporting member may result in making some or all of the embodiments easier to use, since there would be less crowding together of the various members, possibly making operation with one's fingers easier.

In the embodiment shown in fig. 40, the rearward extending portion of the supporting members 7DD include the protrusions 10DD which extend inward from the supporting member 7DD to form a ledge 36DD. The ledge 36DD contacts a rear surface of the hub 3DD. This holds the hollow member 5DD away from the piercing end 11 of the needle 2 against tension from the spring 6. Two releasers 38DD extend rearward from the rear surface of the hub 3DD. The depicted design of the releasers and hub, in combination, would enable them, if formed of plastic, to be formed together in a plastic injection mold without a slide (including a multiple cavity mold).

Twisting of the supporting members 7DD, as with thumb and forefinger, around the outside of the hub 3DD causes the protrusions 10DD on the ends of the supporting members 7DD to contact the slanted sides 37DD of the releasers 38DD. continued twisting in the same direction causes these protrusions 10DD to slide along this slanted surface 37DD of the releasers 38DD, thus pushing the ends of the supporting members 7DD outward until the ledges 36DD are pushed past the outside rear edge of the hub 3DD, releasing the supporting members 7DD from the hub 3DD and thereby allowing the hollow member 5DD to move forward, by force of the spring 6, and to cover the piercing end 11 of the needle 2.

Once past the hub 3DD, the end portion of the supporting members 7DD, including the protrusions 10DD, spring inward so that the ends of the supporting members 7DD are aligned with the frontal surface of the hub 3DD. The hollow member 5DD is thus supported against rearward movement that would reexpose the piercing end 11 of the needle 2.

The embodiment shown in fig. 41 works in the same manner as that shown in fig. 40; However, with the embodiment of fig. 41, the ends of the supporting members 7EE are constructed differently. In this embodiment (of Fig. 41) the outside surface of the end portion of the supporting members 7EE curve inward as they extend rearward so that the end (rear facing) surfaces of the supporting members 7EE do not extend outward past the front outside edge of the hub 3EE when in the safe mode. This design (of Fig. 41) is intended to prevent the ends of the supporting members 7EE from sliding outward, past the front outside edge of the hub 3EE if, while in the safe mode, excessive pressure is exerted against the front of the hollow member in the direction of the hub. Instead, this design should cause the end of the supporting member to slide inward toward the needle 2 as a response to such excessive pressure.

Fig. 42 shows a slight variation of the end of the supporting member 7EE shown in Fig. 41, with the same purpose in mind.

Fig. 43 depicts a supporting member that can be used with the hypodermic needles of Figs. 40 or 41. The protrusion 10FF extending inward from the supporting member 7FF (to form the ledge 36FF) is not located at the end of the supporting member 7FF. This design would allow the exposed needle segment (exposed while in the usable mode) to be longer without increasing the hub length. It is designed to be used with a long neck syringe, as that depicted (in part) in Fig. 23; however, a similar design could be used with a standard syringe where the distance between the protrusion 10FF and the end of the supporting members 7FF is not so great as to prevent proper engagement between the hub and the syringe.

In the embodiment shown in Fig. 44, twisting of the supporting members 7GG, relative to the hub 3GG causes the protrusions 10GG of the supporting members 7GG to contact the slanted sides of the releasers 38GG. These releasers 38GG are shaped like those releasers 38DD shown in Fig. 40, or releasers 38EE shown in Fig. 41. Continued twisting of the supporting members 7GG in the same direction causes the end portions of the supporting members 7GG to bend outward as the protrusions 10GG are urged outward past the rear outer edge of the hub 3GG. This allows the spring 6 to push the hollow member 5GG forward until the protrusions 10GG spring inward into the groove 30GG. Once the protrusions 10GG are in the groove 30GG, the hollow member 5GG is prevented from moving either forward off the end of the needle, or rearward reexposing the piercing end 11 of the needle 2.

The embodiment shown in Fig. 45 works like that shown in Fig. 44 except that, the user rather than a spring pushes the hollow member 5HH forward. Twisting of the supporting members 7HH relative to the hub 3HH first causes the protrusions 10HH to contact the slanted sides of the releasers 38HH, while continued twisting causes the releasers 38HH to urge the protrusions 10HH outward past the outside edge of the hub 3HH. The user then pushes the supporting member 7HH and hollow member 5HH forward (by pushing on either) until the protrusions 10HH spring inward and lodge in the groove 30HH. The hollow member 5HH is thus supported against moving forward or rearward and the piercing end of the needle 11 remains covered by the hollow member 5HH. The releasers 38HH of Fig. 45 are constructed as the releasers 38DD of Fig. 40 or releasers 38EE of Fig. 41.

In the embodiments depicted in Figs. 40, 41, (42, 43), 44 and 45 only one releaser may, if desired, be present on the rear of the hub with only one of the supporting members having the ledge that is engagable with the rear surface of the hub, or with only one supporting member present at all. Also, two releasers may be employed on the rear of the hub with only one supporting member having the ledge engagable with the rear surface of the hub, or only one supporting member present at all.

In the embodiments of Figs. 46, 47 and 48, the supporting members are connected with the hub and extend forward therefrom. The releasers 38II, 38JJ and 38KK of Figs. 46, 47 and 48 respectively are constructed as releasers 38DD or 38EE of Figs. 40 and 41. However, the releasers of these three embodiments are located at the front of the hollow members rather than at the rear of the hubs. In the embodiment depicted in Fig. 46, the user twists the hollow member 5II, as with thumb and forefinger, relative to the supporting members 7II. This causes the releasers 38II to contact the protrusions 10II and eventually urge these protrusions 10II outward past the outer front edge of the hollow member 5II. The spring 6 then pushes the hollow member 5II forward until the ends of the supporting members 7II, including the protrusions 10II spring inward behind the rear of the hollow member 5II. Once the protrusions 10II are lodged behind the hollow member 5II, the hollow member 5II is supported against rearward movement, while the spring 6 which is attached to the hub 3II and the hollow member 5II keeps the hollow member 5II from sliding off the end of the needle 2. It is also noted that the protrusions 10II of this embodiment may, if desired, be replaced with the protrusions 10EE of Fig. 41, or of that depicted in Fig. 42.

Fig. 47 depicts an embodiment where the protrusions 10JJ lodge in a groove 30JJ to support the hollow member 5JJ. As with the embodiment depicted in Fig. 46, twisting of the hollow member 5JJ relative to the supporting members 7JJ causes engagement of the releasers 38JJ with the protrusions 10JJ. With continued twisting, the releasers 38JJ push the protrusions 10JJ outward past the outer edge of the front of the hollow member 5JJ, and the spring 6 pushes the hollow member 5JJ forward until the protrusions 10JJ lodge in the groove 30JJ and thus support the hollow member 5JJ against forward or rearward movement. The spring 6 may or may not be attached to the hub 3JJ and/or the hollow member 5JJ since forward as well as rearward movement of the hollow member 5JJ is prevented.

As with the embodiments of Figs. 46 and 47, in the embodiment depicted in Fig. 48 the protrusions 10KK are pushed outward past the front outer edge of the hollow member 5KK by engagement between the protrusions 10KK and the releasers 38KK, which is accomplished by twisting the hollow member 5KK relative to the supporting members 7KK. However, unlike the previous two embodiments, the user rather than a spring pushes the hollow member 5KK forward. Once the protrusions 10KK lodge in the groove 30KK, the hollow member 5KK is supported against forward or rearward movement.

In the embodiments of Figs. 46, 47, and 48, if so desired, only one releaser may be present on the front of the hollow member with only one of the protrusions having a flat rear surface (ledge), or with only one supporting member present at all. Also, two releasers may be employed on the front of the hollow member with only one protrusion having the flat rear surface or ledge, or with only one supporting member present at all.

Although not shown in the drawings, the hollow members of Figs. 46, 47, and 48 may, if desired, include one or more projections of any of a variety of shapes extending outward therefrom in order to make it easier for those hollow members to be twisted. Such projections may be the shape of a fin, for example, and may also be utilized on other embodiments where the hollow member is released by twisting or pushing, such as those depicted in Figs. 8, 9, 10, 24, 26, 27, 35 or 36.

In the embodiments depicted in Figs. 40 through 48, one or more breakable seals may also be used as between one or both supporting members and the hub (Figs. 40 through 45), or between one or both supporting members and the hollow member (Figs. 46 through 48), or elsewhere. Again, the purpose of such seal(s) would be to help prevent accidental release of the hollow member and/or to assure the user that the hypodermic needle hasn't already been placed in the protective mode once, which would indicate likely contamination. The seals used for this purpose could be like any of those described earlier, such as melted plastic or another melted material, or any other type of adhesive, or the paper or plastic seals described earlier, for example.

In some of those embodiments where the supporting members extend forward from the hub, a modification could be made that would make it possible to have a longer usable needle segment without lengthening the covered portion of the needle. For example, Fig. 3 depicts an embodiment where the supporting members 7A do not extend forward from the front of the hub 3A, but rather extend forward from the rear of the hub 3A. With such a design of the hub and supporting members and with the elimination of the bumps 9A, a hollow member could be used in which the rear portion (of the hollow member) extends rearward over the hub, (as hub 3A of Fig. 3), extending clear back to or nearly to where the supporting members 7A connect with the hub 3A. For example, hollow member 5A of Fig. 3 could flare outward and extend rearward beyond its presently depicted position in the drawing, covering the spring 6 and most of the length of the hub 3A. The protrusions 10A (of Fig. 3) would then lodge behind this lengthened hollow member while in the safe mode. This would allow for the exposed portion of the needle 2 to be longer without necessitating an increase in the length of the covered (covered in the usable mode) portion of the needle. Of course, the spring 6 would have to be of an appropriate length, when extended, to accommodate such a design. Also, the hub may or may not be tapered as hub 3A of Fig. 3 is tapered (The outside of the hub may otherwise be a constant diameter). Releasers, as releasers 38II of Fig. 46 (or depicted more clearly in Fig. 40 as reference character 38DD) could be present on the front of the hollow member 5A to enable it to be released by twisting.

Also, the hollow members depicted in Figs. 6, 9, 10 and 46 could be modified in the same manner, to extend further rearward over the spring 6 and most of the hub, when used with the hub/supporting members combination as just described (as depicted in Fig. 3 without the bumps 9A, with or without a slanted hub). Release of the supporting members could be accomplished in the manner previously described for the embodiments shown in those figures.

Fig. 8 depicts an embodiment where there are no protrusions at the ends at the supporting members 7E. These supporting members 7E could be connected at the rear of the hub 3E, as are supporting members 7A of fig. 3, and the hollow member 5E could be made to extend further rearward, extending over the spring 6 and covering much of the length of the hub 3E in order that this embodiment, as modified, could also have a longer segment of exposed needle.

In those embodiments where the hollow member includes a groove engagable with a protrusion on the supporting members, such hollow members could be made in the manner just described, extending outward (if necessary) and rearward to cover much of the hub in an embodiment where the hub and supporting members are constructed and connected in the manner depicted in Fig. 3 (without the bumps 9A), and where the hub is either tapered (as depicted in Fig. 3) or of a constant diameter. The grooves on the hollow members would also be located further rearward, preferably on that portion of the hollow member that encircles the hub. However, if this is not practical, the groove could encircle the spring. Again, the advantage of this design is that it allows for a longer exposed needle segment to be present without increasing the length of the covered portion of the needle (covered while in the usable mode). This modification is applicable to those embodiments (with a groove in the hollow member) with springs (as those depicted in figs. 35, 36 and 47) as well as those embodiments without springs (as depicted in Figs. 24, 26, 27 and 48). Release of the supporting members could be accomplished in the manner previously described for the embodiments shown in those figures.

It is further disclosed that in all of those embodiments that include a spring and where the supporting members extend forward from the hub, the hollow member could otherwise extend further rearward, over the spring, but only back to or nearly to the hub. This would also allow for a longer exposed needle segment to be present (in the usable mode), without a corresponding increase in the length of the covered portion of the needle. Depending on the particular embodiment so modified, support against rearward movement of the hollow member would either be by alignment of the front of the supporting member(s) and the rear of the hollow member, or otherwise by positioning of a protrusion on the supporting member(s) in a groove in the hollow member. When a groove in the hollow member is used, that would also be positioned further rearward. Such a modification could be applied to those embodiments shown in Figs. 3, 5, 6 , 7, 8, 9, 10, 35, 36, 46 and 47, for example.

One or more breakable seals may also be used in all such embodiments with a lengthened hollow member (as just described) in order to help prevent accidental release of the hollow member and/or to assure the user that the hypodermic needle hasn't already been used. Where applicable, the seal(s) may also be used as the means to hold the hollow member away from the piercing end of the needle. Such seal(s) could be as any of those previously described for these purposes as applied to other embodiments. The location of such a seal(s) could be between the hollow member and the hub or supporting member, for example. Also, in any of these embodiments only one supporting member may be used, if so desired. And further, a projection of some type, (as the "fin" described earlier, for example) may be present on the hollow member to make it easier to twist or push those hollow members to release them.

The following applies to all of the embodiments that utilize springs, and where support means against rearward movement of the hollow member is achieved by alignment of the end(s) of the supporting member(s) and the frontal surface of the hub: the springs may be such a length so that when the hypodermic needle is in the safe mode (the spring is extended) there is a space between the end(s) of the supporting member(s) and the front of the hub, or, alternately, the end(s) of the supporting member(s) may be in contact with the front of the hub while in the safe mode. In the ladder case, forward motion of the springs, hollow members and supporting members, after release, causes the spring to over extend itself so that the end(s) of the supporting member(s) clear the hub, and then spring back to make contact with the front of the hub.

Likewise, where support means is achieved of the end(s) of the supporting member(s) and the rear of the hollow member, and where springs may either be of such a length so that, when extended (the hypodermic needle is in the safe mode), the end(s) of the supporting member(s) are in contact with the rear of the hollow member, or, alternately, there may be a space between the end(s) of the supporting member (s) and the rear of the hollow member. Again, in the former situation, the forward momentum of the extending spring and the hollow member causes overextension of the spring, allowing the hollow member to clear the end(s) of the supporting member(s) before recoil of the spring pulls the hollow member back, to make contact with the end(s) of the supporting member(s).

In any of the embodiments described herein, a small cord or cable or the like, hereafter referred to as a "cord" may be used to connect the hub with the hollow member (or supporting member) in order to prevent the hollow member from sliding off the end of the needle. This cord so employed would be particularly applicable in those embodiments where there is no protrusion on a supporting member that lodges in a groove or depression in the the hub. In those embodiments where springs are used, this cord could be used to prevent an overextending spring from pushing the hollow member off the end of the needle. The cord could also be used to eliminate the need to connect the spring to the hub and the hollow member (or supporting member) in those embodiments where such a connection is otherwise necessary. Furthermore, such a cord could be used in place of a spring in all of the embodiments that have been shown and/or described as having a spring (again, especially in those embodiments where no protrusion on a supporting member lodges in a groove or depression in the hub. In such an application, the user would push the hollow member forward.

Health workers sometimes use a small disposable device in which to prick a finger in order to take a single drop of blood for a sample. A device called a lancet is used for this purpose. The various embodiments of this invention are adaptable to such a device, or a device designed for such a function. Of course, a hollow needle capable of transmitting fluids need not be used in this function, nor would a hub designed to transmit fluids to or from a syringe. To perform such a function, a round sharply pointed needle that is not necessarily hollow, or a flat sharply pointed blade-like member may be attached to a handle with the essential outer characteristics of the various hubs described herein. All of the other features described for the various embodiments, and variations of those embodiments, would be used in adapting any one embodiment, or variation thereof, to this function; again, all features except the fluid transmitting means of the needle and hub. Of course, certain modifications of the various members may be necessary. For example, if a flat blade-like member is used as the piercing member, the hole through the hollow member would have a conforming shape. If a spring is used to push the hollow member forward, that spring should, preferably, have a conforming shape around the periphery of that blade-like member.

The hole passing through the hollow members of the various embodiments may be of varying character, as those depicted in figures 32, 33 and 34. Figures 32, 33 and 34 are cross-sectional views of hollow members with the needle 2 included. The structures depicted in figures 33 or 34, or a similar grooved construction, may prove advantageous in the manufacture of the hollow members in some or all of the embodiments, especially when those hollow members are formed of plastic.

The invention herein described, including all embodiments and variations of those embodiments, and including the various functions thereof, can be used in either human or veterinary medicine for use on either humans or animals.

Also, where a hypodermic needle and a syringe are combined as a single unit, any of the embodiments of hypodermic needles, or variations thereof herein described, may be packaged and/or sold in such a combination with a syringe as a single unit.

While several embodiments and modifications thereto of the invention have been shown and described herein as best modes for carrying out the invention, it should be understood that changes and modifications may be made thereto without departing from the subject matter coming within the scope of the invention and the following claims.

## Claims

1. A safety hypodermic needle comprising:
a) a needle (2) provided with a passageway there through for the transmission of fluids, said needle being sharpened at its distal end to form a piercing end (11);
b) a hub (3h, 3i, 3j, 3k, 3l, 3m, 3n, 3p, 3q, 3u, 3w, 3x, 3aa, 3bb, 3cc, 3DD, 3EE, 3GG or 3HH) connected with the proximal end of said needle, said hub being engageable with a syringe so that fluid may be transmitted between said syringe and said needle, passing through said needle;
c) a hollow member (5h, 5k, 51, 5m, 5p, 5u, 5aa, 5bb, 5cc, 5DD, 5EE, 5GG or 5HH) including a hole or a groove therein, said hollow member being positioned over a portion of said needle so that said needle passes into or through said hole or said groove;
d) at least one supporting member (7h, 7i, 7j, 7k, 70k, 7l, 70l, 7m, 7p, 7q, 7u, 7v, 7w, 7x, 70cc, 7DD, 7EE, 7FF, 7GG or 7HH) connected with said hollow member, said supporting member including a rearward extending portion adapted to engage said hub;
**characterised by** the provision of:
e) a releasable securement means (18h; 23 with 22i plus 18i; 18j; 25 with 3k plus 18k; 1001 with 3l plus 18l; 18m; 18p; 18q; 18u; 32v in 30v; 32w with 3w; 33 with 34; 18aa; 100bb with 3bb plus 18bb; 100cc with 3cc plus 18cc; 36DD with 3DD; 36EE with 3EE; 36FF; 10GG with 3GG; or 10HH with 3HH) for holding said hollow member away from the piercing end of said needle, so that after said hollow member is released by said securement means, said hollow member may be moved forward, along said needle, to a position at least partially covering said piercing end of said needle, with means provided (10h with 3h; 10i with 3i; 10j with 3j; 100k and 10k with 3k; 100l and 101 with 3l; 7m with 3m; 10p with 3p; 10q with 3q; 10u in 30u; 7v with 30v; 10aa in 30aa; 10bb and 100bb with 29bb at 30bb; 10cc and 100cc with 29cc at 30cc; 10DD with 3DD; 10EE with 3EE; 10GG in 30GG; or 10HH in 30HH) for said rearward extending portion of said supporting member to engage said hub to support said hollow member against movement back toward said hub that would reexpose said piercing end of said needle.

2. The safety hypodermic needle of claim 1, and further including a spring (6) positioned between said hollow member and said hub, so that after said hollow member is released by said securement means, said hollow member will move forward along said needle, by force of said spring, to said position at least partially covering said piercing end of said needle.

3. The safety hypodermic needle of claim 2, wherein said releasable securement means includes:
a) a ledge (36DD, 36EE or 36FF) located on said rearward extending portion of said supporting member, said ledge being engageable with a rear surface of said hub to hold said hollow member away from the piercing end of said needle;
b) at least one releasor (38DD, 38EE or 38GG) extending rearward from said hub, said releasor being engageable with said supporting member so that said supporting member can be released from said hub by turning or rotating said supporting member relative to said hub.

4. The safety hypodermic needle of claims 2 or 3, wherein two supporting members extend rearward from said hollow member.

5. The safety hypodermic needle of claims 2, 3 or 4, wherein said support means is provided by the end or ends of said one or more supporting members being adapted to push against the frontal surface of said hub.

6. The safety hypodermic needle of claim 2, wherein said rearward extending portion of said one or more supporting members curve inward (as does 7m), toward the axial center of said hypodermic needle, as said one or more supporting members extend rearward away from said hollow member.

7. The safety hypodermic needle of claim 6, wherein said support means is provided by the end or ends of said one or more supporting members adapted to push against the frontal surface of said hub.

8. The safety hypodermic needle of claims 6 or 7, wherein two supporting members extend rearward from said hollow member.

9. The safety hypodermic needle of claims 6, 7 or 8, wherein said one or more supporting members and said hollow member are plastic and are formed together as a single piece in a plastic injection mold without a slide.

10. The safety hypodermic needle of claims 2, 3 or 4, wherein said support means is provided by a protrusion (10aa; 10bb and 100bb; 10cc and 100cc or 10GG) at or near the end of said one or more supporting members positioned in a groove or depression (30aa, 30bb, 30cc or 30GG) in said hub.

11. The safety hypodermic needle of claim 1, wherein said releasable securement means includes:
a) a ledge (36DD; 36EE or 36FF) located on said rearward extending portion of said supporting member, said ledge being engageable with a rear surface of said hub to hold said hollow member away from the piercing end of said needle;
b) at least one releasor (38DD, 38EE, 38GG or 38HH) extending rearward from said hub, said releasor being engageable with said supporting member so that said supporting member can be released from said hub by turning said supporting member relative to said hub.

12. The safety hypodermic needle of claims 1 or 11, wherein two supporting members extend rearward from said hollow member.

13. The safety hypodermic needle of claims 1, 11 or 12, wherein said support means is provided by a protrusion (10u; 10aa; 10bb and 100bb; 10cc and 100cc; 10GG or 10HH) at or near the end of said one or more supporting members positioned in a groove or depression (30u, 30v, 30aa, 30bb, 30cc, 30GG or 30HH) in said hub.

14. A safety hypodermic needle comprising:
a) a needle (2) provided with a passageway there through for the transmission of fluids, said needle being sharpened at its distal end to form a piercing end (11);
b) a hub (3a, 3b, 3c, 3d, 3e, 3r, 3y, 3z, 3ll, 3JJ or 3KK) connected with the proximal end of said needle, said hub being engageable with a syringe so that fluid may be transmitted between said syringe and said needle, passing through said needle;
c) a hollow member (5a, 5b, 5c, 5d, 5e, 5f, 5g, 5r, 5s, 5t, 5y, 5z, 5ll, 5JJ or 5KK) including a hole or a groove therein, said hollow member being positioned over a portion of said needle so that said needle passes into or through said hole or said groove;
d) at least one supporting member (7a, 7b, 7c, 7d, 7e, 7f, 7g, 7r, 7s, 7II, 7JJ or 7KK) connected with said hub, said supporting member including a forward extending portion adapted to engage said hollow member;
**characterised by** the provision of:
e) a releasable securement means (10a with 5a; 10b with 5b; 10c with 5c; 10d with 5d; 18e; 10f with 19f plus 18f; 10g with 19g; 18r; 10s with 19s plus 18s; 18t; 18y; 10z with 19z plus 18z; 10II with 5ll; 10JJ with 5JJ; or 10KK with 5KK) for holding said hollow member away from the piercing end of said needle, so that after said hollow member is released by said securement means, said hollow member may be moved forward, along said needle, to a position at least partially covering said piercing end of said needle, with means provided (10a with 5a; 10b with 5b; 10c with 5c; 10d with 5d; 7e with 5e; 10f with 5f; 10r in 30r; 10s in 30s; 10t in 30t; 10y in 30y; 10z in 30z; 10II with 5ll; 10JJ in 30JJ; or 10KK in 30KK) for said forward extending portion of said supporting member to engage said hollow member to support said hollow member against movement back toward said hub that would reexpose said piercing end of said needle.

15. The safety hypodermic needle of claim 14, and further including a spring (6) positioned between said hollow member and said hub so that after said hollow member is released by said securement means, said hollow member will move forward along said needle, by force of said spring, to said position at least partially covering said piercing end of said needle.

16. The safety hypodermic needle of claim 15, wherein said releasable securement means includes:
a) a ledge (rear of 10II; rear of 10JJ;) located on said forward extending portion of said supporting member, said ledge being engageable with a front surface of said hollow member to hold said hollow member away from the piercing end of said needle;
b) at least one releasor (38ll, 38JJ) extending forward from said hollow member, said releasor being engageable with said supporting member so that said hollow member can be released from said supporting member by turning or rotating said hollow member relative to said supporting member.

17. The safety hypodermic needle of claims 15 or 16, wherein two supporting members extend forward from said hub.

18. The safety hypodermic needle of claims 15, 16 or 17, wherein said support means is provided by the end or ends of said one or more supporting members being adapted to push against a rear surface of said hollow member.

19. The safety hypodermic needle of claim 15, wherein said forward extending portion of said one or more supporting members curve inward (as does 7e), toward the axial center of said hypodermic needle, as said one or more supporting members extend forward away from said hub.

20. The safety hypodermic needle of claim 19, wherein said support means is provided by the end or ends of said one or more supporting members being adapted to push against a rear surface of said hollow member.

21. The safety hypodermic needle of claims 19 or 20, wherein two supporting members extend forward from said hub.

22. The safety hypodermic needle of claims 19, 20 or 21, wherein said one or more supporting members and said hub are plastic and are formed together as a single piece in a plastic injection mold without a slide.

23. The safety hypodermic needle of claims 15, 16 or 17, wherein said support means is provided by a protrusion (10y, 10z, 10JJ) at or near the end of one or more supporting members positioned in a groove or depression (30y, 30z, 30JJ) in said hollow member.

24. The safety hypodermic needle of claim 14, wherein said releasable securement means includes:
a) a ledge located on said forward extending portion of said supporting member, said ledge being engageable with a front surface of said hollow member to hold said hollow member away from the piercing end of said needle;
b) at least one releasor (38ll, 38JJ or 38KK) extending forward from said hollow member, said releasor being engageable with said supporting member so that said hollow member can be released from said supporting member by turning said hollow member relative to said supporting member.

25. The safety hypodermic needle of claims 14 or 24, wherein two supporting members extend forward from said hub.

26. The safety hypodermic needle of claims 14, 24 or 25, wherein said support means is provided by a protrusion (10r, 10s, 10t, 10y, 10z, 10JJ or 10KK) at or near the end of said one or more supporting members positioned in a groove or depression (30s, 30t, 30y, 30z, 30JJ or 30KK) in said hollow member.

27. The safety hypodermic needle of any of the proceeding claims, in combination with a syringe.

## Patentansprüche

1. Hypodermatische Nadel mit Sicherungsvorrichtung, die folgende Teile umfaßt:
a) eine Kanüle (2) mit einer Durchtrittsöffnung zur Leitung von Flüssigkeiten, wobei die Nadel an ihrem distalen Ende zur Bildung eines Einstichendes (11) zugespitzt ist;
b) einen Ansatz (3h, 3i, 3j, 3k, 31, 3m, 3n, 3p, 3q, 3u, 3w, 3x, 3aa, 3bb, 3cc, 3DD, 3EE, 3GG oder 3HH), der mit dem proximalen Ende der Nadel verbunden ist, wobei der Ansatz mit einer Spritze in Eingriff zu bringen ist, so daß zwischen der Spritze und der Nadel Flüssigkeit befördert werden kann, die durch die Nadel fließt;
c) einen Hohlkörper (5h, 5k, 5l, 5m, 5p, 5u, 5aa, 5bb, 5cc, 5DD, 5EE, 5GG oder 5HH) mit einem darin ausgebildeten Loch oder einem Kanal, wobei der Hohlkörper über einem Teil der Nadel angeordnet ist, so daß sich die Nadel in das Loch oder den Kanal hinein- oder durch sie hindurcherstreckt;
d) mindestens ein Halteglied (7h, 7i, 7j, 7k, 70k, 7l, 70l, 7m, 7p, 7q, 7u, 7v, 7w, 7x, 70cc, 7DD, 7EE, 7FF, 7GG oder 7HH), das mit dem Hohlkörper verbunden ist, wobei das Halteglied einen sich nach hinten erstreckenden Teil aufweist, der zur Eingriffsverbindung mit dem Ansatz ausgebildet ist;
**gekennzeichnet durch** das Vorhandensein
e) einer lösbaren Sicherungsvorrichtung (18h; 23 mit 22i und 18i; 18j; 25 mit 3k und 18k; 100l mit 3l und 18l; 18m; 18p; 18q; 18u; 32v in 30v; 32w mit 3w; 33 mit 34; 18aa; 100bb mit 3bb und 18bb; 100cc mit 3cc und 18cc; 36DD mit 3DD; 36EE mit 3EE; 36FF; 10GG mit 3GG; oder 10HH mit 3HH), um den Hohlkörper zu dem Einstichende der Nadel auf Abstand zu halten, so daß nach Freigabe des Hohlkörpers **durch** die Sicherungsvorrichtung der Hohlkörper an der Nadel entlang nach vorn in eine Position bewegt werden kann, in der er das Einstichende der Nadel zumindest teilweise bedeckt, wobei an dem sich nach hinten erstreckenden Teil des Haltegliedes Vorrichtungen vorhanden sind (10h mit 3h; 10i mit 3i; 10j mit 3j; 100k und 10k mit 3k; 100l und 10l mit 31; 7m mit 3m; 10p mit 3p; 10q mit 3q; 10u in 30u; 7v mit 30v; 10aa in 30aa; 10bb und 100bb mit 29bb bei 30bb; 10cc und 100cc mit 29cc bei 30cc; 10DD mit 3DD; 10EE mit 3EE; 10GG in 30GG; oder 10HH in 30HH), um mit dem Ansatz in Eingriff zu treten und den Hohlkörper gegen eine Bewegung zurück zu dem Ansatz zu sichern, wodurch das Einstichende der Nadel wieder freigelegt würde.

2. Hypodermatische Nadel mit Sicherungsvorrichtung nach Anspruch 1,
ferner mit einer Feder (6), die zwischen dem Hohlkörper und dem Ansatz angeordnet ist, so daß sich der Hohlkörper nach seiner Freigabe durch die Sicherungsvorrichtung durch die Kraft der Feder entlang der Nadel nach vorn in die Position bewegt, in der er zumindest teilweise das Einstichende der Nadel abdeckt.

3. Hypodermatische Nadel mit Sicherungsvorrichtung nach Anspruch 2,
bei der die lösbare Sicherungsvorrichtung folgende Teile enthält:
a) einen Anschlag (36DD, 36EE oder 36FF), der an dem sich nach hinten erstreckenden Teil des Haltegliedes angeordnet ist, wobei der Anschlag mit einer hinteren Fläche des Ansatzes in Eingriff zu bringen ist, um den Hohlkörper von dem Einstichende der Nadel auf Abstand zu halten;
b) mindestens eine sich von dem Ansatz nach hinten erstreckende Lösevorrichtung (38DD, 38EE oder 38GG), wobei die Lösevorrichtung mit dem Halteglied in Eingriff zu bringen ist, so daß das Halteglied von dem Ansatz durch Drehen oder Rotieren des Haltegliedes relativ zu dem Ansatz gelöst werden kann.

4. Hypodermatische Nadel mit Sicherungsvorrichtung nach den Ansprüchen 2 oder 3,
bei der sich zwei Halteglieder von dem Hohlkörper nach hinten erstrecken.

5. Hypodermatische Nadel mit Sicherungsvorrichtung nach den Ansprüchen 2, 3 oder 4,
bei der die Haltevorrichtung durch das oder die Enden des oder der Halteglieder gebildet ist, die derart ausgebildet sind, daß sie gegen die vordere Fläche des Ansatzes drücken.

6. Hypodermatische Nadel mit Sicherungsvorrichtung nach Anspruch 2,
bei der der sich nach hinten erstreckende Teil des oder der Halteglieder (wie 7m) nach innen in Richtung auf die axiale Mitte der hypodermatischen Nadel gekrümmt ist, wobei sich das oder die Halteglieder von dem Hohlkörper weg nach hinten erstrecken.

7. Hypodermatische Nadel mit Sicherungsvorrichtung nach Anspruch 6,
bei der die Haltevorrichtung durch das oder die Enden des oder der Halteglieder gebildet ist, die derart ausgebildet sind, daß sie gegen die vordere Fläche des Ansatzes drücken.

8. Hypodermatische Nadel mit Sicherungsvorrichtung nach den Ansprüchen 6 oder 7,
bei der sich zwei Halteglieder von dem Hohlkörper nach hinten erstrecken.

9. Hypodermatische Nadel mit Sicherungsvorrichtung nach den Ansprüchen 6, 7 oder 8,
bei der das oder die Halteglieder und der Hohlkörper aus Kunststoff bestehen und einstückig in einer Kunststoff-Spritzgußform ohne Schieber hergestellt werden.

10. Hypodermatische Nadel mit Sicherungsvorrichtung nach den Ansprüchen 2, 3 oder 4,
bei der die Haltevorrichtung durch einen Vorsprung (10aa; 10bb und 100bb; 10cc und 100cc oder 10GG) an oder nahe dem Ende des oder der Halteglieder gebildet wird, der in einer Nut oder Vertiefung (30aa, 30bb, 30cc oder 30GG) in dem Ansatz positioniert wird.

11. Hypodermatische Nadel mit Sicherungsvorrichtung nach Anspruch 1,
bei der die lösbare Sicherungsvorrichtung folgende Teile enthält:
a) einen Anschlag (36DD, 36EE oder 36FF), der an dem sich nach hinten erstreckenden Teil des Haltegliedes angeordnet ist, wobei der Anschlag mit einer hinteren Fläche des Ansatzes in Eingriff zu bringen ist, um den Hohlkörper von dem Einstichende der Nadel auf Abstand zu halten;
b) mindestens eine sich von dem Ansatz nach hinten erstreckende Lösevorrichtung (38DD, 38EE, 38GG oder 38HH), wobei die Lösevorrichtung mit dem Halteglied in Eingriff zu bringen ist, so daß das Halteglied von dem Ansatz durch Drehen des Haltegliedes relativ zu dem Ansatz gelöst werden kann.

12. Hypodermatische Nadel mit Sicherungsvorrichtung nach den Ansprüchen 1 oder 11,
bei der sich zwei Halteglieder von dem Hohlkörper nach hinten erstrecken.

13. Hypodermatische Nadel mit Sicherungsvorrichtung nach den Ansprüchen 1, 11 oder 12,
bei der die Haltevorrichtung durch einen Vorsprung (10u; 10aa; 10bb und 100bb; 10cc und 100cc; 10GG oder 10HH) an oder nahe dem Ende des oder der Halteglieder gebildet wird, der in einer Nut oder Vertiefung (30u, 30v, 30aa, 30bb, 30cc, 30GG oder 30HH) in dem Ansatz positioniert wird.

14. Hypodermatische Nadel mit Sicherungsvorrichtung, die folgende Teile umfaßt:
a) eine Kanüle (2) mit einer Durchtrittsöffnung zur Leitung von Flüssigkeiten, wobei die Nadel an ihrem distalen Ende zur Bildung eines Einstichendes (11) zugespitzt ist;
b) einen Ansatz (3a, 3b, 3c, 3d, 3e, 3r, 3y, 3z, 3II, 3JJ oder 3KK), der mit dem proximalen Ende der Nadel verbunden ist, wobei der Ansatz mit einer Spritze in Eingriff zu bringen ist, so daß zwischen der Spritze und der Nadel Flüssigkeit befördert werden kann, die durch die Nadel fließt;
c) einen Hohlkörper (5a, 5b, 5c, 5d, 5e, 5f, 5g, 5r, 5s, 5t, 5y, 5z, 5II, 5JJ oder 5KK) mit einem darin ausgebildeten Loch oder einem Kanal, wobei der Hohlkörper über einem Teil der Nadel angeordnet ist, so daß sich die Nadel in das Loch oder den Kanal hinein- oder durch sie hindurcherstreckt;
d) mindestens ein Halteglied (7a, 7b, 7c, 7d, 7e, 7f, 7g, 7r, 7s, 7II, 7JJ oder 7KK), das mit dem Ansatz verbunden ist, wobei das Halteglied einen sich nach vorn erstreckenden Teil aufweist, der zur Eingriffsverbindung mit dem Hohlkörper ausgebildet ist;
**gekennzeichnet durch** das Vorhandensein
e) einer lösbaren Sicherungsvorrichtung (10a mit 5a; 10b mit 5b; 10c mit 5c; 10d mit 5d; 18e; 10f mit 19f und 18f; 10g mit 19g; 18r; 10s mit 19s und 18s; 18t; 18y; 10z mit 19z und 18z; 10II mit 5II; 10JJ mit 5JJ; oder 10KK mit 5KK), um den Hohlkörper zu dem Einstichende der Nadel auf Abstand zu halten, so daß nach Freigabe des Hohlkörpers **durch** die Sicherungsvorrichtung der Hohlkörper an der Nadel entlang nach vorn in eine Position bewegt werden kann, in der er das Einstichende der Nadel zumindest teilweise abdeckt, wobei an dem sich nach vorn erstreckenden Teil des Haltegliedes Vorrichtungen vorhanden sind (10a mit 5a; 10b mit 5b; 10c mit 5c; 10d mit 5d; 7e mit 5e; 10f mit 5f; 10r in 30r; 10s in 30s; 10t in 30t; 10y in 30y; 10z in 30z; 10II mit 5II; 10JJ in 30JJ; oder 10KK in 30KK), um mit dem Hohlkörper in Eingriff zu treten und den Hohlkörper gegen eine Bewegung zurück zu dem Ansatz zu sichern, wodurch das Einstichende der Nadel wieder freigelegt würde.

15. Hypodermatische Nadel mit Sicherungsvorrichtung nach Anspruch 14,
ferner mit einer Feder (6), die zwischen dem Hohlkörper und dem Ansatz angeordnet ist, so daß sich der Hohlkörper nach seiner Freigabe durch die Sicherungsvorrichtung durch die Kraft der Feder entlang der Nadel nach vorn in die Position bewegt, in der er zumindest teilweise das Einstichende der Nadel abdeckt.

16. Hypodermatische Nadel mit Sicherungsvorrichtung nach Anspruch 15,
bei der die lösbare Sicherungsvorrichtung folgende Teile enthält:
a) einen Anschlag (Rückseite von 10II; Rückseite von 10JJ), der an dem sich nach vorn erstreckenden Teil des Haltegliedes angeordnet ist, wobei der Anschlag mit einer vorderen Fläche des Hohlkörpers in Eingriff zu bringen ist, um den Hohlkörper von dem Einstichende der Nadel auf Abstand zu halten;
b) mindestens eine sich von dem Hohlkörper nach vorn erstreckende Lösevorrichtung (38II, 38JJ), wobei die Lösevorrichtung mit dem Halteglied in Eingriff zu bringen ist, so daß der Hohlkörper von dem Halteglied durch Drehen oder Rotieren des Hohlkörpers relativ zu dem Halteglied gelöst werden kann.

17. Hypodermatische Nadel mit Sicherungsvorrichtung nach den Ansprüchen 15 oder 16,
bei der sich zwei Halteglieder von dem Ansatz nach vorn erstrecken.

18. Hypodermatische Nadel mit Sicherungsvorrichtung nach den Ansprüchen 15, 16 oder 17,
bei der die Haltevorrichtung durch das oder die Enden des oder der Halteglieder gebildet ist, die derart ausgebildet sind, daß sie gegen eine hintere Fläche des Hohlkörpers drücken.

19. Hypodermatische Nadel mit Sicherungsvorrichtung nach Anspruch 15,
bei der der sich nach vorn erstreckende Teil des oder der Halteglieder (wie 7e) nach innen in Richtung auf die axiale Mitte der hypodermatischen Nadel gekrümmt ist, wobei sich das oder die Halteglieder von dem Ansatz weg nach vorn erstrecken.

20. Hypodermatische Nadel mit Sicherungsvorrichtung nach Anspruch 19,
bei der die Haltevorrichtung durch das oder die Enden des oder der Halteglieder gebildet ist, die derart ausgebildet sind, daß sie gegen eine hintere Fläche des Hohlkörpers drücken.

21. Hypodermatische Nadel mit Sicherungsvorrichtung nach den Ansprüchen 19 oder 20,
bei der sich zwei Halteglieder von dem Ansatz nach vorn erstrecken.

22. Hypodermatische Nadel mit Sicherungsvorrichtung nach den Ansprüchen 19, 20 oder 21,
bei der das oder die Halteglieder und der Ansatz aus Kunststoff bestehen und einstückig in einer Kunststoff-Spritzgußform ohne Schieber hergestellt werden.

23. Hypodermatische Nadel mit Sicherungsvorrichtung nach den Ansprüchen 15, 16 oder 17,
bei der die Haltevorrichtung durch einen Vorsprung (10y, 10z, 10JJ) an oder nahe dem Ende des oder der Halteglieder gebildet wird, der in einer Nut oder Vertiefung (30y, 30z, 30JJ) in dem Hohlkörper positioniert wird.

24. Hypodermatische Nadel mit Sicherungsvorrichtung nach Anspruch 14,
bei der die lösbare Sicherungsvorrichtung folgende Teile enthält:
a) einen Anschlag, der an dem sich nach vorn erstreckenden Teil des Haltegliedes angeordnet ist, wobei der Anschlag mit einer vorderen Fläche des Hohlkörpers in Eingriff zu bringen ist, um den Hohlkörper von dem Einstichende der Nadel auf Abstand zu halten;
b) mindestens eine sich von dem Hohlkörper nach vorn erstreckende Lösevorrichtung (38II, 38JJ oder 38KK), wobei die Lösevorrichtung mit dem Halteglied in Eingriff zu bringen ist, so daß der Hohlkörper von dem Halteglied durch Drehen des Hohlkörpers relativ zu dem Halteglied gelöst werden kann.

25. Hypodermatische Nadel mit Sicherungsvorrichtung nach den Ansprüchen 14 oder 24,
bei der sich zwei Halteglieder von dem Ansatz nach vorn erstrecken.

26. Hypodermatische Nadel mit Sicherungsvorrichtung nach den Ansprüchen 14, 24 oder 25,
bei der die Haltevorrichtung durch einen Vorsprung (10r, 10s, 10t, 10y, 10z, 10JJ oder 10KK) an oder nahe dem Ende des oder der Halteglieder gebildet wird, der in einer Nut oder Vertiefung (30s, 30t, 30y, 30z, 30JJ oder 30KK) in dem Hohlkörper positioniert wird.

27. Hypodermatische Nadel mit Sicherungsvorrichtung nach einem der vorhergehenden Ansprüche in Kombination mit einer Spritze.

## Revendications

1. Aiguille hypodermique de sécurité comprenant :
a) une aiguille (2) pourvue d'un passage qui la traverse pour la transmission de fluides, ladite aiguille étant effilée à son extrémité distale pour former une extrémité de percement (11);
b) un moyeu (3h, 3i, 3j, 3k, 3l, 3m, 3n, 3p, 3q, 3u, 3w, 3x, 3aa, 3bb, 3cc, 3DD, 3EE, 3GG ou 3HH) raccordé à l'extrémité proximale de ladite aiguille, ledit moyeu pouvant être engagé sur une seringue de sorte qu'un fluide puisse être transmis entre ladite seringue et ladite aiguille, en passant à travers ladite aiguille;
c) un élément creux (5h, 5k, 5l, 5m, 5p, 5u, 5aa, 5bb, 5cc, 5DD, 5EE, 5GG ou 5HH) comprenant un trou ou une rainure, ledit élément creux étant positionné sur une partie de ladite aiguille de telle sorte que ladite aiguille passe dans ledit trou ou ladite rainure ou à travers celui-ci ou celle-ci;
d) au moins un élément de support (7h, 7i, 7j, 7k, 70k, 7l, 70l, 7m, 7p, 7q, 7u, 7v, 7w, 7x, 70cc, 7DD, 7EE, 7FF, 7GG ou 7HH) raccordé audit élément creux, ledit élément de support comprenant une partie s'étendant vers l'arrière et qui est à même de s'engager sur ledit moyeu;
**caractérisée en ce qu'**on met en oeuvre :
e) un moyen de fixation libérable (18h; 23 avec 22i plus 18i; 18j; 25 avec 3k plus 18k; 100l avec 3l plus 181; 18m; 18p; 18q; 18u; 32v dans 30v; 32w avec 3w; 33 avec 34; 18aa; 100bb avec 3bb plus 18bb; 100cc avec 3cc plus 18cc; 36DD avec 3DD; 36EE avec 3EE; 36FF; 10GG avec 3GG; ou 10HH avec 3HH) pour maintenir ledit élément creux dégagé de l'extrémité de percement de ladite aiguille afin que, une fois que ledit élément creux est libéré par ledit moyen de fixation, ledit élément creux puisse être déplacé en avant, le long de ladite aiguille, jusque dans une position recouvrant au moins partiellement ladite extrémité de percement de ladite aiguille, avec des moyens prévus (10h avec 3h; 10i avec 3i; 10j avec 3j; 100k et 10k avec 3k; 100l et 10l avec 3l; 7m avec 3m; 10p avec 3p; 10q avec 3q; 10u dans 30u; 7v avec 30v; 10aa dans 30aa; 10bb et 100bb avec 29bb à 30bb; 10cc et 100cc avec 29cc à 30cc; 10DD avec 3DD; 10EE avec 3EE; 10GG dans 30GG; ou 10HH dans 30HH) pour que ladite partie s'étendant vers l'arrière dudit élément de support s'engage sur ledit moyeu pour supporter ledit élément creux à l'encontre de tout déplacement de reflux vers ledit moyeu qui ré-exposerait ladite extrémité de percement de ladite aiguille.

2. Aiguille hypodermique de sécurité selon la revendication 1, comprenant en outre un ressort (6) disposé entre ledit élément creux et ledit moyeu de sorte que, une fois que ledit élément creux est libéré par ledit moyen de fixation, ledit élément creux se déplace en avant le long de ladite aiguille, sous la force dudit ressort, jusque dans ladite position où il recouvre au moins partiellement ladite extrémité de percement de ladite aiguille.

3. Aiguille hypodermique de sécurité selon la revendication 2, dans laquelle ledit moyen de fixation libérable comprend :
a) un rebord (36DD, 36EE ou 36FF) situé sur ladite partie s'étendant vers l'arrière dudit élément de support, ledit rebord pouvant s'engager sur une surface arrière dudit moyeu pour maintenir ledit élément creux dégagé de l'extrémité de percement de ladite aiguille;
b) au moins un déclencheur (38DD, 38EE ou 38GG) s'étendant vers l'arrière dudit moyeu, ledit déclencheur étant à même de s'engager sur ledit élément de support de telle sorte que ledit élément de support puisse être libéré dudit moyeu par rotation dudit élément de support par rapport audit moyeu.

4. Aiguille hypodermique de sécurité selon la revendication 2 ou 3, dans laquelle deux éléments de support s'étendent vers l'arrière dudit élément creux.

5. Aiguille hypodermique de sécurité selon la revendication 2, 3 ou 4, dans laquelle ledit moyen de support est formé par la ou les extrémités desdits un ou plusieurs éléments de support qui sont adaptés pour pousser contre la surface frontale dudit moyeu.

6. Aiguille hypodermique de sécurité selon la revendication 2, dans laquelle ladite partie s'étendant vers l'arrière desdits un ou plusieurs éléments de support s'incurve vers l'intérieur (comme l'élément 7m), vers le centre axial de ladite aiguille hypodermique, lorsque lesdits un ou plusieurs éléments de support s'étendent vers l'arrière à l'opposé dudit élément creux.

7. Aiguille hypodermique de sécurité selon la revendication 6, dans laquelle ledit moyen de support est formé par la ou les extrémités desdits un ou plusieurs éléments de support adaptées pour pousser contre la surface frontale dudit moyeu.

8. Aiguille hypodermique de sécurité selon la revendication 6 ou 7, dans laquelle deux éléments de support s'étendent vers l'arrière dudit élément creux.

9. Aiguille hypodermique de sécurité selon la revendication 6, 7 ou 8, dans laquelle lesdits un ou plusieurs éléments de support et ledit élément creux sont en matière plastique et sont formés conjointement sous la forme d'une pièce unique dans un moule à injection de matière plastique sans coulisseau.

10. Aiguille hypodermique de sécurité selon la revendication 2, 3 ou 4, dans laquelle ledit moyen de support est formé par une saillie (10aa; 10bb et 100bb; 10cc et 100cc ou 10GG) qui se trouve à l'extrémité ou à proximité de l'extrémité desdits un ou plusieurs éléments de support positionnés dans une rainure ou une dépression (30aa, 30bb, 30cc ou 30GG) dudit moyeu.

11. Aiguille hypodermique de sécurité selon la revendication 1, dans laquelle ledit moyen de fixation libérable comprend :
a) un rebord (36DD; 36EE ou 36FF) situé sur ladite partie s'élevant vers l'arrière dudit élément de support, ledit rebord étant à même de s'engager sur une surface arrière dudit moyeu pour maintenir ledit élément creux dégagé de ladite extrémité de percement de ladite aiguille;
b) au moins un déclencheur (35DD, 38EE, 38GG ou 38HH) s'étendant vers l'arrière dudit moyeu, ledit déclencheur étant à même de s'engager sur ledit élément de support de sorte que ledit élément de support puisse être libéré dudit moyeu en faisant tourner ledit élément de support par rapport audit moyeu.

12. Aiguille hypodermique de sécurité selon la revendication 1 ou 11, dans laquelle deux éléments de support s'étendent vers l'arrière dudit élément creux.

13. Aiguille hypodermique de sécurité selon la revendication 1, 11 ou 12, dans laquelle ledit moyen de support est formé par une saillie (10u; 10aa; 10bb et 100bb; 10cc et 100cc; 10GG ou 10HH) à l'extrémité ou à proximité de l'extrémité desdits un ou plusieurs éléments de support positionnés dans une rainure ou dans un creux (30u, 30v, 30aa, 30bb, 30cc, 30GG ou 30HH) dans ledit moyeu.

14. Aiguille hypodermique de sécurité comprenant :
a) une aiguille (2) pourvue d'un passage qui la traverse pour la transmission de fluides, ladite aiguille étant effilée à son extrémité distale pour former une extrémité de percement (11);
b) un moyeu (3a, 3b, 3c, 3d, 3e, 3r, 3y, 3z, 3ll, 3JJ ou 3KK) raccordé à l'extrémité proximale de ladite aiguille, ledit moyeu étant à même de s'engager sur une seringue de sorte qu'un fluide puisse être transmis entre ladite seringue et ladite aiguille en passant à travers ladite aiguille;
c) un élément creux (5a, 5b, 5c, 5d, 5e, 5f, 5g, 5r, 5s, 5t, 5y, 5z, 5ll, 5JJ ou 5KK) comprenant un orifice ou une rainure, ledit élément creux étant disposé sur une partie de ladite aiguille de sorte que ladite aiguille passe dans ou à travers ledit trou ou ladite rainure;
d) au moins un élément de support (7a, 7b, 7c, 7d, 7e, 7f, 7g, 7r, 7s, 7ll, 7JJ ou 7KK), raccordé audit moyeu, ledit élément de support comprenant une partie s'étendant vers l'avant qui est à même de s'engager sur ledit élément creux;
**caractérisé par** la présence des éléments suivants :
e) un moyen de fixation libérable (10a avec 5a; 10b avec 5b; 10c avec 5c; 10d avec 5d; 18e; 10f avec 19f plus 18f; 10g avec 19g; 18r; 10s avecl9s plus 18s; 18t; 18y; 10z avec 19z plus 18z; 1011 avec 511; 10JJ avec 5JJ; ou 10KK avec 5KK) pour maintenir ledit élément creux dégagé de l'extrémité de percement de ladite aiguille de sorte que, une fois que ledit élément creux est libéré par ledit moyen de fixation, ledit élément creux puisse être déplacé vers l'avant, le long de ladite aiguille, jusque dans une position où il recouvre au moins partiellement ladite extrémité de percement de ladite aiguille, avec des moyens prévus (10a avec 5a; 10b avec 5b; 10c avec 5c; 10d avec 5d; 7e avec 5e; 10f avec 5f; 10r dans 30r; 10s dans 30s; 10t dans 30t; 10y dans 30y; 10z dans 30z; 1011 avec 511; 10JJ dans 30JJ; ou 10KK dans 30KK) pour que ladite partie s'étendant vers l'avant dudit élément de support s'engage dans ledit élément creux afin de supporter ledit élément creux à l'encontre de tout mouvement de reflux vers ledit moyeu, qui ré-exposerait ladite extrémité de percement de ladite aiguille.

15. Aiguille hypodermique de sécurité selon la revendication 14, comprenant en outre un ressort (6) disposé entre ledit élément creux et ledit moyeu de telle sorte que, une fois que ledit élément creux est libéré par ledit moyen de fixation, ledit élément creux se déplace vers l'avant le long de ladite aiguille, sous l'action dudit ressort, jusque dans ladite position où il recouvre au moins partiellement ladite extrémité de percement de ladite aiguille.

16. Aiguille hypodermique de sécurité selon la revendication 15, dans laquelle ledit moyen de fixation libérable comprend :
a) un rebord (arrière de 10ll; arrière de 10JJ) situé sur ladite partie s'étendant vers l'avant dudit élément de support, ledit rebord étant à même de s'engager sur une surface frontale dudit élément creux pour maintenir ledit élément creux dégagé de l'extrémité de percement de ladite aiguille;
b) au moins un déclencheur (38ll, 38JJ) s'étendant vers l'avant dudit élément creux, ledit déclencheur étant à même de s'engager sur ledit élément de support de sorte que ledit élément creux puisse être libéré dudit élément de support par rotation dudit élément creux par rapport audit élément de support.

17. Aiguille hypodermique de sécurité selon la revendication 15 ou 16, dans laquelle deux éléments de support s'étendent vers l'avant dudit moyeu.

18. Aiguille hypodermique de sécurité selon la revendication 15, 16 ou 17, dans laquelle ledit moyen de support est formé par la ou les extrémités desdits un ou plusieurs éléments de support adaptées à pousser contre une surface arrière dudit élément creux.

19. Aiguille hypodermique de sécurité selon la revendication 15, dans laquelle ladite partie s'étendant vers l'avant desdits un ou plusieurs éléments de support s'incurve à l'intérieur (comme l'élément 7e), vers le centre axial de ladite aiguille hypodermique, lorsque lesdits un ou plusieurs éléments de support s'étendent en avant à l'opposé dudit moyeu.

20. Aiguille hypodermique de sécurité selon la revendication 19, dans laquelle ledit moyen de support est formé par la ou les extrémités desdits un ou plusieurs éléments de support adaptées pour pousser contre une surface arrière dudit élément creux.

21. Aiguille hypodermique de sécurité selon la revendication 19 ou 20, dans laquelle deux éléments de support s'étendent vers l'avant dudit moyeu.

22. Aiguille hypodermique de sécurité selon la revendication 19, 20 ou 21, dans laquelle lesdits un ou plusieurs éléments de support et ledit moyeu sont en matière plastique et sont formés conjointement sous la forme d'une seule pièce dans un moule à injection de matière plastique sans coulisseau.

23. Aiguille hypodermique de sécurité selon la revendication 15, 16 ou 17, dans laquelle ledit moyen de support est formé par une saillie (10y, 10z, 10JJ) à l'extrémité ou à proximité de l'extrémité d'un ou plusieurs éléments de support positionnés dans une rainure ou un creux (30y, 30z, 30JJ) dudit élément creux.

24. Aiguille hypodermique de sécurité selon la revendication 14, dans laquelle ledit moyen de fixation libérable comprend :
a) un rebord situé sur ladite partie s'étendant vers l'avant dudit élément de support, ledit rebord étant à même de s'engager sur une surface frontale dudit élément creux pour maintenir ledit élément creux dégagé de l'extrémité de percement de ladite aiguille;
b) au moins un déclencheur (38ll, 38JJ ou 38KK) s'étendant vers l'avant dudit élément creux, ledit déclencheur étant à même de s'engager sur ledit élément de support de telle sorte que ledit élément creux puisse être libéré dudit élément de support en faisant tourner ledit élément creux par rapport audit élément de support.

25. Aiguille hypodermique de sécurité selon la revendication 14 ou 24, dans laquelle deux éléments de support s'étendent vers l'avant dudit moyeu.

26. Aiguille hypodermique de sécurité selon la revendication 14, 24 ou 25, dans laquelle ledit moyen de support est formé par une saillie (10r, 10s, 10t, 10y, 10z, 10JJ ou 10KK) qui se trouve à l'extrémité ou à proximité de l'extrémité desdits un ou plusieurs éléments de support positionnés dans une rainure ou un creux (30s, 30t, 30y, 30z, 30JJ ou 30KK) dans ledit élément creux.

27. Aiguille hypodermique de sécurité selon l'une quelconque des revendications précédentes, en combinaison avec une seringue.
